# EUROPÄISCHE PATENTSCHRIFT

(11) **EP 2 594 493 B1**
(45) Veröffentlichungstag und Bekanntmachung des Hinweises auf die Patenterteilung: **23.03.2016**
(21) Anmeldenummer: 12193687.6
(22) Anmeldetag: 21.11.2012
(51) Int. Cl.: B65B 55/08, B65B 59/00, A61L 2/08, B29C 49/42

(54) **Vorrichtung zur Innen- und Außensterilisierung von Vorformlingen mittels Ladungsträgerstrahlen sowie entsprechendes Verfahren und Behandlungsanlage**
Device for internal and external sterilisation of preforms by means of charge carrier rays and corresponding method and handling system
Dispositif de stérilisation intérieure et extérieure de préforms au moyen du rayonnement d'un support de chargement et méthod et ligne de traitement correspondant

(30) Priorität: 21.11.2011 DE 102011055553
(43) Veröffentlichungstag der Anmeldung: 22.05.2013
(73) Patentinhaber: Krones AG, 93073 Neutraubling (DE)
(72) Erfinder: Knott, Josef, 93073 Neutraubling (DE); Frankenberger, Günter, 93073 Neutraubling (DE); Engelhard, Patrick, 93073 Neutraubling (DE); Krüger, Jochen, 93073 Neutraubling (DE); Söllner, Jürgen, 93073 Neutraubling (DE); Müller, Holger, 93073 Neutraubling (DE); Watter, Martin, 93073 Neutraubling (DE); Scheuren, Hans, 93073 Neutraubling (DE); Laumer, Roland, 93073 Neutraubling (DE)
(74) Vertreter: Bittner, Bernhard

(56) Entgegenhaltungen:
- EP-A1- 2 316 495
- WO-A1-2010/090247
- DE-A1-102007 050 582
- DE-A1-102008 038 143
- DE-A1-102009 041 215
- DE-A1-102010 012 569
- US-A- 3 527 017

## Beschreibung

Die Erfindung betrifft eine Vorrichtung zum Sterilisieren zumindest eines Abschnitts einer Innenwandung und eines Abschnitts einer Außenwandung von Vorformlingen mittels beschleunigter Ladungsträger mit mindestens einer ersten Außenbeaufschlagungseinrichtung zum Sterilisieren zumindest eines Abschnitts einer Außenwandung von Vorformlingen und einer Innenbeaufschlagungseinrichtung zum Sterilisieren zumindest eines Abschnitts einer Innenwandung von Vorformlingen, wobei die Innenbeaufschlagungseinrichtung zumindest abschnittsweise durch eine Öffnung in den Vorformling einführbar ist, um eine Innenwandung des Vorformlings mit austretenden Ladungsträgern zu beaufschlagen, mit einer Transporteinrichtung, mittels welcher die Vorformlinge während deren Sterilisation entlang eines Transportpfades transportierbar sind.

Weiterhin betrifft die Erfindung eine Anlage zum Behandeln von Vorformlingen, welche mindestens eine Vorrichtung zum Sterilisieren zumindest eines Abschnitts einer Innenwandung und eines Abschnitts einer Außenwandung von Vorformlingen mittels beschleunigter Ladungsträger mit mindestens einer ersten Außenbeaufschlagungseinrichtung zum Sterilisieren zumindest eines Abschnitts einer Außenwandung von Vorformlingen und einer Innenbeaufschlagungseinrichtung zum Sterilisieren zumindest eines Abschnitts einer Innenwandung von Vorformlingen aufweist, sowie ein Verfahren zum Sterilisieren zumindest eines Abschnitts einer Innenwandung und eines Abschnitts einer Außenwandung von Vorformlingen mittels beschleunigter Ladungsträger,
wobei der Vorformling durch mindestens eine erste Außenbeaufschlagungseinrichtung zum Sterilisieren zumindest eines Abschnitts einer Außenwandung von Vorformlinger und eine Innenbeaufschlagungseinrichtung zum Sterilisieren zumindest eines Abschnitts einer Innenwandung von Vorformlinger mit Ladungsträgern beaufschlagt wird, wobei die Innenbeaufschlagungseinrichtung zumindest abschnittsweise durch eine Öffnung in das Behältnis eingeführt wird, um eine Innenwandung des Vorformlings mit austretenden Ladungsträgern zu beaufschlagen, wobei die Vorformlinge während deren Sterilisation mit einer Transporteinrichtung entlang eines Transportpfades transportiert werden.

Die Sterilisation eines zu befüllenden Behälters ist neben dem eigentlichen Füllvorgang einer der zentralen Prozessschritte bei einer aseptischen Abfüllung. Die möglichen Sterilisationsformen variieren hinsichtlich der Entkeimungsmittel und der Prozessführung. Allen gemein ist jedoch, dass die abtötende Wirkung aufgrund chemischer Prozesse bewirkt wird. So ist es beispielsweise bekannt, die Innenwandung von Behältnissen mit Dampf oder Wasserstoffperoxid zu sterilisieren. Derartige Verfahren, wie sie z.B. aus der WO 2010/090247 bekannt sind, sind jedoch mit Nachteilen verbunden, da es durch die Behandlung beispielsweise mit Wasserstoffperoxid zu einer Aufweichung des Materials kommen kann. Um diese Nachteile vermeiden zu können, nutzen neuere Entwicklungen ionisierende Strahlung um eine Keimreduzierung zu erreichen. Diese Strahlung besteht in den meisten Anwendungen aus beschleunigten Elektronen, die in einer entsprechenden Anlage erzeugt und auf den bzw. in den zu sterilisierenden Behälter geleitet werden. Daraus ergibt sich eine Verringerung oder vollständige Vermeidung des Einsatzes chemischer Stoffe und unter anderem eine Reduzierung der Beschaffungs- und Entsorgungskosten.

Die Begriffe "Behältnis", "Behälter" und "Kunststoffbehältnis" werden im Folgenden zur Vereinfachung synonym verwendet. Dabei schließen diese Begriffe auch Vorprodukte derartiger Behältnisse ein. Insbesondere beziehen sich diese Begriffe auf Flaschen, bevorzugt Getränkeflaschen. Erfindungsgemäß beziehen sich diese Begriffe, insbesondere "Behältnisse", nur auf Vorformlinge (z.B. Kunststoffvorformlinge für z.B. Flaschen).

Außerdem werden die Begriffe Desinfektion und Sterilisation bzw. Desinfektions- und Sterilisationsmittel synonym verwendet.

Als Reinraum wird ein gegenüber der Umgebung abgeschlossener Bereich verstanden, in dem gegenüber der Umgebung veränderte Bedingungen in Bezug auf die Sterilität aufrecht erhalten werden können. Insbesondere wird als Reinraum ein Bereich verstanden, der bestimmte Mindestanforderungen in Bezug auf eine maximale Verunreinigung mit Keimen erfüllt. Bevorzugt erfüllt jeder Volumenanteil des Reinraums und jede der inneren Oberflächen des Reinraums Mindestanforderungen in Bezug auf Sterilität.

Aus dem Stand der Technik sind Systeme zur Sterilisation bekannt, welche aus einer Elektronenerzeugungsvorrichtung und einer Bündelungseinrichtung bestehen. Dabei sind sowohl Systeme bekannt, welche die Behältnisse von außen oder von innen sterilisieren. Bei der Innensterilisation werden außerhalb des zu sterilisierenden Behältnisses erzeugte Ladungsträger durch verschiedene z.B. mechanische oder elektronische Elemente in das zu sterilisierende Behältnis gelenkt. In diesem bildet sich eine Wolke aus Elektronen welche durch Wechselwirkungen mit etwaigen unerwünschten Mikroorganismen diese inaktiviert.

Ein Beispiel einer solchen Einrichtung zur Innensterilisation eines Behälters mittels Elektronen ist in der DE 198 82 252 T1 beschrieben. Dabei ist eine Elektronenstrahlquelle vorgesehen, die die Strahlung von außerhalb in das Innere des Behältnisses richtet.

Aus der Druckschrift WO 97/07024 A ist ein Verfahren bekannt, in dem zumindest Teile der Elektronenquelle in das Behältnisinnere eingeführt werden können. Es ist ein Verfahren zum Reinigen bzw. Sterilisieren von Produktverpackungen mittels Elektronenstrahl beschrieben. Die in WO 97/07024 A offenbarte Vorrichtung umfasst eine teilweise in das Behältnisinnere einführbare Elektronenkanone welche beschleunigte Elektronen in das Innere eines Behältnisses einbringt. Ein parallel dazu eingeleiteter Strom eines mit dem Elektronenstrahl wechselwirkenden Gases dient entweder zur Ablenkung des Elektronenstrahls in Richtung des Gasstroms oder aber durch Ionisierung des Gases als Hilfsstoff zu Sterilisierung.

Aus der DE 102 010 012 569 ist eine Vorrichtung zum Sterlisieren von Behältnissen mit separaten Einrichtungen zur Innen- und Außersterilisation bekannt.

Weiter ist aus der DE 102 008 038 143 eine Vorrichtung zum Sterilisieren von Vorformlingen mit einer Abstandsänderungseinrichtung bekannt.

Die DE 102 007 050 582 zeigt zuden eine Vorrichtung nach dem Oberbegrift des Anspruchs 1 bzw. ein Verfahren nach den Oberbegrift des Ansrpruchs 13.

Wird keine separate Innensterilisation der Behältnisse vorgenommen, sind deutlich leistungsstärkere Ladungsträgererzeuger notwendig, da die beschleunigten Ladungsträger zusätzlich mindestens eine Außenwandung des Behältnisses durchdringen müssen. Dementsprechend starke Abschirmungen der Umgebung gegen erzeugte (Streu-) Strahlung ist bei derartigen Vorrichtungen notwendig.

Besonders vorteilhaft wäre es, die Vorteile einer Innensterilisation mit denen einer separaten Sterilisation der Behältnisaußenwandung verbinden zu können. Derartige Vorrichtungen, die die Vorteile der jeweiligen Sterilisationsmethode mit denen der anderen verbinden können, sind jedoch aus dem Stand der Technik nicht bekannt. Dies beruht insbesondere darauf, dass die Behandlungszeiten für die verschiedenen Sterilisationen unterschiedlich sind und die jeweilige Transporteinrichtung damit sehr unterschiedliche Rahmenbedingungen erfüllen muss.

Der Erfindung liegt somit die Aufgabe zugrunde, eine Vorrichtung zum Sterilisieren zumindest eines Abschnitts einer Innenwandung und eines Abschnitts einer Außenwandung von Vorformlingen mittels beschleunigter Ladungsträger mit separaten Einrichtungen zur Innensterilisation und Außensterilisation bereitzustellen, die eine Vielzahl von Vorformlingen in einem kontinuierlichen Prozess behandeln kann.

Da insbesondere in den zur Produktion und Befüllung von Behältnissen verwendeten Anlagen hohe Taktraten und Durchsatzzahlen erreicht werden, ist es weiterhin Aufgabe der Erfindung eine entsprechende Anlage zum Behandeln von Behältnissen bereitzustellen, welche eine derartige Vorrichtung zum Sterilisieren von Vorformlingen einschließt.

Darüber hinaus ist es Aufgabe der Erfindung, ein Verfahren zum Sterilisieren zumindest eines Abschnitts einer Innenwandung und eines Abschnitts einer Außenwandung von Vorformlingen mittels beschleunigter Ladungsträger mit separaten Einrichtungen zur Innensterilisation und Außensterilisation bereitzustellen, mittels welchem eine Vielzahl von Vorformlingen in einem kontinuierlichen Prozess behandelt werden kann.

Dies wird erfindungsgemäß durch eine Vorrichtung nach Anspruch 1, eine Anlage nach Anspruch 12 und ein Verfahren nach Anspruch 13 erreicht. Vorteilhafte Ausführungsformen und Weiterbildungen sind Gegenstand der Unteransprüche.

Ein wesentlicher Aspekt der Erfindung ist eine Vorrichtung zum Sterilisieren zumindest eines Abschnitts einer Innenwandung und eines Abschnitts einer Außenwandung von Vorformlingen mittels beschleunigter Ladungsträger mit mindestens einer ersten Außenbeaufschlagungseinrichtung zum Sterilisieren zumindest eines Abschnitts einer Außenwandung von Vorformlingen und einer Innenbeaufschlagungseinrichtung zum Sterilisieren zumindest eines Abschnitts einer Innenwandung von Vorformlingen, wobei die Innenbeaufschlagungseinrichtung zumindest abschnittsweise durch eine Öffnung in den Vorformling einführbar ist, um eine Innenwandung des Vorformlings mit austretenden Ladungsträgern zu beaufschlagen, mit einer Transporteinrichtung, mittels welcher die Vorformlinge während deren Sterilisation entlang eines Transportpfades transportierbar sind, wobei entlang eines Abschnitts des Transportpfades der Vorformlinge zwischen der ersten Außenbeaufschlagungseinrichtung und der Innenbeaufschlagungseinrichtung eine Abstandsänderungseinrichtung, zur Veränderung eines Abstands zwischen zwei entlang des Transportpfades aufeinander folgender Vorformlinge (d.h. die Teilung) angeordnet ist.

Mit einer derartigen Vorrichtung ist es möglich, die Innensterilisation und die Außensterilisation von Vorformlingen in einer einzigen gemeinsamen Vorrichtung durchzuführen. Gleichzeitig ist es möglich, die Vorrichtung besonders kompakt auszuführen, da die Abschirmung der Umgebung gegenüber der in der Vorrichtung entstehenden Strahlung der jeweiligen in den verschiedenen Beaufschlagungseinrichtungen entstehenden Strahlung angepasst werden kann. So könnte die Abschirmung im Bereich der Außenbeaufschlagungseinrichtung beispielsweise stärker ausgeführt sein als diejenige im Bereich der Innenbeaufschlagungseinrichtung. Die Außenbeaufschlagungseinrichtung und die Innenbeaufschlagungseinrichtung sind als separate Elemente ausgeführt und räumlich voneinander getrennt.

Bevorzugt befindet sich die Außenbeaufschlagungseinrichtung stromaufwärts der Innenbeaufschlagungseinrichtung. Es wäre jedoch auch denkbar, dass die Innenbeaufschlagungseinrichtung stromaufwärts der Außenbeaufschlagungseinrichtung angeordnet ist.

Die Innenbeaufschlagungseinrichtung ist bevorzugt in Form eines in das Behältnisinnere einführbaren Strahlfinger oder Fingeremitters ausgeführt. Bevorzugt befinden sich eine Vielzahl derartiger Strahlfinger auf einer gemeinsamen Transporteinrichtung, z.B. einem Transportstern.

Die Anordnung einer Abstandsänderungseinrichtung, zur Veränderung eines Abstands zwischen zwei entlang des Transportpfades aufeinander folgender Behältnisse in einem Abschnitt des Transportpfades zwischen der ersten Außenbeaufschlagungseinrichtung und der Innenbeaufschlagungseinrichtung, ermöglicht es, die jeweiligen Transportelemente, die die Behältnisse während des jeweiligen Sterilisationsprozesses transportieren, unabhängig voneinander auf die jeweiligen Anforderungen anzupassen. So könnte es beispielsweise vorteilhaft sein, dass während der Außensterilisation die Behältnisse vergleichsweise eng aufeinander folgend transportiert werden. Insbesondere kann bei Behältnissen, insbesondere bei Vorformlingen der Abstand zweier entlang des Transportpfades direkt aufeinander folgender Behältnisse < 10 cm, bevorzugt < 7,5 cm, besonders bevorzugt < 5 cm sein. Diese enge Anordnung ist insbesondere beim Transport und bei der Sterilisation von Vorformlingen möglich, welche deutlich geringere Abmessungen, insbesondere einen geringeren Durchmesser, aufweisen als fertige Behältnisse. Durch die enge Folge von Vorformlingen kann im Bereich der Außenbeaufschlagungseinrichtung die Größe der zwischen zwei aufeinander direkt folgenden Behältnisse befindlichen Lücke minimiert sein. Dadurch kann auch der Anteil an Ladungsträgern, welche die zu sterilisierenden Behältnisse durch diese Lücke hindurch ungenutzt passiert, minimiert werden.

Bevorzugt ist es durch die zumindest abschnittsweise enge Folge von direkt benachbarten Behältnissen möglich, die Geschwindigkeit der einzelnen Behältnisse entlang des Transportpfades in diesem Bereich zu verringern und dabei die Anzahl der pro Zeitintervall transportierten Behältnisse konstant zu halten. Dadurch wird vorzugsweise im Bereich der Außenbeaufschlagungseinrichtung eine längere Behandlungsdauer erreicht, die z.B. eine Drehung des Behältnisses, bevorzugt eine vollständige Drehung des Behältnisses, entlang dessen Längsachse während der Ladungsträgerbeaufschlagung ermöglicht.

Demgegenüber können abschnittsweise größere Abstände zwischen entlang des Transportpfades direkt aufeinander folgender Behältnisse vorteilhaft sein. Beispielsweise ist es möglich, dass im Bereich der Innenbeaufschlagungseinrichtung größere Abstände benachbarter Behältnisse notwendig sind, da die Innenbeaufschlagungseinrichtung teilweise in das Behältnis eingeführt wird. Die Innenbeaufschlagungseinrichtung weist dabei zumindest abschnittsweise einen Durchmesser auf, der das Einführen dieses Abschnitts in das Innere des Behältnisses ermöglicht. Andere Abschnitte der Innenbeaufschlagungseinrichtung wie z.B. eine Ladungsträgererzeugungseinrichtung oder eine Ladungsträgerbeschleunigungseinrichtung, eine Stromversorgungseinrichtung, eine Transformatoreinrichtung, eine Kühleinrichtung oder andere können dabei jedoch unabhängig voneinander auch größere Dimensionen aufweisen. Dadurch und evtl. auch durch den Mechanismus, mittels welchem die Relativbewegung zwischen der Innenbeaufschlagungseinrichtung und dem zu sterilisierenden Behältnis entlang dessen Längsachse realisiert wird, könnten sehr geringe Abstände zwischen entlang des Transportpfades direkt aufeinander folgender Behältnisse nachteilig sein. Bevorzugt ist daher ein Abstand zweier entlang des Transportpfades direkt aufeinander folgender Behältnisse in einem Abschnitt des Transportpfades, bevorzug im Bereich der Innenbeaufschlagungseinrichtung > 5 cm, bevorzugt > 7,5 cm, besonders bevorzugt > 10 cm.

Bevorzugt ist eine Ausführungsform der Vorrichtung zum Sterilisieren von Vorformlingen, insbesondere Vorformlinge für (d.h. zum Herstellen von) Behältnisse für Getränke und/oder andere fluide Medien. Dies ermöglicht durch die im Vergleich zu fertigen Behältnissen deutlich geringere Ausdehnung (bzw. Größe, Länge und/oder Durchmesser) der Vorformlinge eine geringere zu sterilisierende Oberfläche. Dadurch ist es auch möglich, die Beaufschlagungseinrichtungen kleiner zu dimensionieren. Bevorzugt lassen sich auch Strahlungsmenge, Strahlungsintensität, Beschleunigungsspannung und/oder andere Parameter verbessern, so dass der Energiebedarf der Vorrichtung und/oder deren Herstell- bzw. Anschaffungskosten reduziert werden können.

Bevorzugt ist eine Ausführungsform der Vorrichtung zum Sterilisieren von Behältnissen bei der der Transportpfad zumindest in einem Abschnitt, in welchem die erste Außenbeaufschlagungseinrichtung zum Sterilisieren zumindest eines Abschnitts einer Außenwandung von Behältnissen angeordnet ist, gekrümmt ist. Durch diese Ausführungsform ist es möglich, Transporteinrichtungen wie z.B. Transportsterne zu verwenden, bei welchen der Transportpfad zumindest abschnittsweise im Wesentlichen mäanderförmig ausgebildet ist. Diese Ausführungsform ermöglicht weiterhin eine besonders kompakte Bauweise.

Bevorzugt ist vorgesehen, dass in der Vorrichtung zum Sterilisieren von Behältnissen auf der der ersten Außenbeaufschlagungseinrichtung gegenüberliegenden Seite des Transportpfades eine weitere Außenbeaufschlagungseinrichtung angeordnet ist, wobei die Außenbeaufschlagungseinrichtung und die weitere Außenbeaufschlagungseinrichtung besonders bevorzugt entlang des Transportpfades versetzt angeordnet sind.

Diese Ausführungsform mit zwei sich bezüglich des Transportpfads gegenüberliegenden Außenbeaufschlagungseinrichtungen ist vorteilhaft, da so auch ohne eine Rotation der Behältnisse um deren eigene Längsachse die Beaufschlagung des Behältnisses mit Ladungsträgern von beiden Seiten möglich ist. Die Transporteinrichtung kann daher deutlich vereinfacht werden und z.B. auf einen Mechanismus zur vollständigen Rotation der Behältnisse und deren Längsachse verzichtet werden. Sollte trotz der gegenüberliegenden Außenbeaufschlagungseinrichtungen die Sterilisation in den Randbereichen dennoch nicht ausreichend sein, ist eine Schwenkbewegung der Behältnisse denkbar. Dabei soll unter einer Schwenkbewegung im Wesentlichen eine Rotation des Behältnisses um Bruchteile einer vollständigen Umdrehung verstanden werden. Die dafür notwendige Steuerung ist wesentlich einfacher als die für vollständige Rotationen und gewährleistet dennoch die ausreichende Sterilisierung aller äußeren Oberflächenbereiche des Behältnisses.

Um zu vermeiden, dass Ladungsträger direkt aufeinander zu beschleunigt werden, was u.a. zu besonders hoher Ladungsträgerdichte im Überlappungsbereich der emittierten Ladungsträgerwolken oder zu Beschädigungen der Außenbeaufschlagungseinrichtungen durch die ständige Beaufschlagung von Ladungsträgern, welche von der gegenüberliegenden Außenbeaufschlagungseinrichtung in deren Richtung emittiert werden, führen könnte, sind die Außenbeaufschlagungseinrichtungen bevorzugt um einen Winkel zwischen 2 und 20°, besonders bevorzugt zwischen 5 und 10°, eines Krümmungskreises des Transportpfads, versetzt entlang des Transportpfades angeordnet.

Bevorzugt sind somit die Maxima der Ladungsträgerwolken, welche von den Außenbeaufschlagungseinrichtungen in Richtung des Transportpfades abgegeben werden, mindestens 2 cm, bevorzugt mindestens 5 cm, besonders bevorzugt mindestens 10 cm und bevorzugt höchstens 200 cm, bevorzugt höchstens 100 cm, besonders bevorzugt höchstens 50 cm, entlang der Transportrichtung der Behältnisse beabstandet angeordnet. Dadurch gelangen die Behältnisse während des Transports zunächst in den Einflussbereich einer Außenbeaufschlagungseinrichtung und werden von dieser mit Ladungsträgern beaufschlagt. Zu einem späteren Zeitpunkt, wenn das Behältnis in einen stromabwärts befindlichen Einflussbereich der anderen Außenbeaufschlagungseinrichtung gelangt, wird es von dieser mit Ladungsträgern beaufschlagt. Dabei können sich die Einflussbereiche der beiden Außenbeaufschlagungseinrichtungen überlappen.

Bevorzugt ist mindestens eine Außenbeaufschlagungseinrichtung im Wesentlichen außerhalb eines Reinraums angeordnet. Als im Wesentlichen außerhalb des Reinraums soll in diesem Zusammenhang verstanden werden, dass ein Großteil der Außenbeaufschlagungseinrichtung von Außen und ohne Öffnung des Reinraums - und damit ohne mögliche Kontamination des Reinraums - zugänglich ist, das Ladungsträgeraustrittsfenster jedoch in einer Wandung des Reinraums angeordnet ist, so dass die Ladungsträger aus der Außenbeaufschlagungseinrichtung in den Reinraum und auf das zu sterilisierende Behältnis emittiert werden können.

Vorteilhaft ist die Vorrichtung zum Sterilisieren von Behältnissen in einem Bereich des Transportpfades, in welchem die erste Außenbeaufschlagungseinrichtung angeordnet ist, zumindest abschnittsweise von einer Strahlungsabschirmungsvorrichtung mit mindestens einer äußeren Strahlungsabschirmungseinrichtung und einer inneren Strahlungsabschirmungseinrichtung umgeben, durch welche eine von der ersten Außenbeaufschlagungseinrichtung abgegebene Strahlung zumindest teilweise absorbierbar ist.

Die Ausführungsform mit zwei Strahlungsabschirmungseinrichtungen erlaubt es, einen Transportkanal auszubilden, welcher sich zwischen den beiden Strahlungsabschirmungseinrichtungen erstreckt und in welchem die Behältnisse transportiert werden können. Als innere Strahlungsabschirmungseinrichtung wird diesbezüglich eine Strahlungsabschirmungseinrichtung verstanden, welche Ladungsträger in Richtung einer Innenseite der Vorrichtung abschirmt.

Da sich in diesem Bereich vorteilhafterweise keine Personen aufhalten, kann diese Strahlungsabschirmungseinrichtung evtl. weniger stark ausgebildet sein als eine äußere Strahlungsabschirmungseinrichtung. Diese schirmt bevorzugt auch die Umgebung, in der sich evtl. auch - zumindest temporär- Personen aufhalten können, von den Ladungsträgern und/oder der Strahlung ab. Bei der bevorzugten Ausführungsform der Anordnung der Außenbeaufschlagungseinrichtungen entlang eines Abschnitts eines Kreisumfangs ist die innere Strahlungsabschirmungseinrichtung bezüglich des Transportpfades und eines Kreismittelpunkts radial innenliegend und die äußere Strahlungsabschirmungseinrichtung bezüglich des Transportpfades und eines Kreismittelpunkts radial außenliegend.

Die äußere Strahlungsabschirmungseinrichtung ist demnach bevorzugt auf einer radial außenliegenden Seite eines gekrümmten Transportpfades angeordnet. Da die Außenbeaufschlagungseinrichtungen bevorzugt die stärksten Ladungsträgererzeugungseinrichtungen darstellen, ist in deren Einflussbereich eine besonders starke Abschirmung notwendig. Bevorzugt weist die in diesem Bereich angeordnete Strahlungsabschirmungseinrichtung daher stärkere Abschirmungseigenschaften auf als eine Strahlungsabschirmungseinrichtung, welche entlang eines anderen Bereiches des Transportpfades angeordnet ist.

Durch die Krümmung des Transportpfades im Bereich der Außenbeaufschlagungseinrichtungen ist es möglich, dass durch die äußere Strahlungsabschirmungseinrichtung Strahlung und/oder Ladungsträger - bevorzugt mindestens zweifach, besonders bevorzugt mehrfach - reflektiert, bevorzugt in Richtung der zu sterilisierenden Behältnisse reflektiert bzw. umgelenkt werden.

Bevorzugt ist eine Vorrichtung zum Sterilisieren von Behältnissen bei der die Strahlungsabschirmungsvorrichtung einen während des Transports von Behältnissen entlang des Transportpfades stationären Anteil und einen anderen, während des Transports von Behältnissen entlang des Transportpfades gegenüber dem ersten Anteil relativbeweglichen Anteil, aufweist.

Durch diese Ausführungsvariante ist es möglich, dass Greif- bzw. Halteelemente der Bewegung des relativbeweglichen Anteils der Strahlungsabschirmungsvorrichtung folgen und sich (zumindest mit einer Vektorkomponente) parallel zu der Transportrichtung der Behältnisse bewegen.

Vorteilhaft ist die Geschwindigkeit des relativbeweglichen Anteils der Strahlungsabschirmungsvorrichtung zumindest in einem Abschnitt des Transportpfades auf eine Geschwindigkeit, mit der die Behältnisse entlang des Transportpfades bewegt werden, anpassbar.

Bevorzugt durchdringen die Greif- bzw. Halteelemente den relativbeweglichen Anteil der Strahlungsabschirmungsvorrichtung zumindest abschnittsweise, so dass ein Anteil der Greif- bzw. Halteelemente im Inneren des Transportkanals angeordnet ist und dort die Behältnisse greifen bzw. halten kann. Ein anderer Anteil der Greif- bzw. Halteeinrichtung ist bevorzugt außerhalb des Transportkanals angeordnet. Dieser außerhalb liegende Anteil kann beispielsweise wartungsintensive Komponenten aufweisen, so dass deren Wartung möglich ist, ohne den sterilen Transportkanal öffnen zu müssen. Bevorzugt befinden sich Elemente eines Hub-, Dreh-, Schwenk- und/oder Transportmechanismus außerhalb des sterilen Transportkanals.

Insbesondere bei der Behandlung von Vorformlingen ist es notwendig, den Hubmechanismus auf die geringen Dimensionen der Vorformlinge im Vergleich zu fertigen Behältnissen anzupassen.

Bevorzugt werden die Behältnisse zumindest im Bereich der Außensterilisation durch eine im Wesentlichen einteilige Spannhülse gehalten bzw. transportiert.

Bevorzugt ist eine Vorrichtung zum Sterilisieren von Behältnissen bei der die Innenbeaufschlagungseinrichtung zum Sterilisieren zumindest eines Abschnitts einer Innenwandung von Behältnissen eine Beschleunigungseinrichtung mit einer geringeren Beschleunigungsspannung aufweist als eine Beschleunigungseinrichtung der ersten Außenbeaufschlagungseinrichtung.

Die Innenbeaufschlagungseinrichtung muss zumindest teilweise in das Innere des Behältnisses eingeführt werden. Daher bestehen besondere Anforderungen an deren Dimensionierung, die starke Abschirmungen, große Transformatoren, voluminöse Kühlelemente und andere große Bauteile nachteilig machen. Insbesondere bei der Innensterilisation von Vorformlingen besteht aufgrund deren geringen Ausdehnung kein Bedarf an besonders stark beschleunigten Ladungsträgern. Oft weisen Vorformlinge einen Innendurchmesser von < 5 cm, < 3 cm oder sogar < 2 cm auf, so dass die Ladungsträger keine großen Distanzen zwischen einem im Behältnisinneren befindlichen Ladungsträgeraustrittsfenster und der Innenoberfläche des Behältnisses zurücklegen müssen. Daher sind für die Innenbeaufschlagungseinrichtung in diesem Fall geringe Beschleunigungsspannungen ausreichend. Eine Reduzierung der Beschleunigungsspannung auf das erforderliche Maß bietet Vorteile in der Energieeffizienz, den Betriebskosten und den Aufwendungen für eine Ausreichende Abschirmung der Umgebung gegenüber Ladungsträgern und/oder Strahlung.

Demgegenüber emittiert die Außenbeaufschlagungseinrichtung Ladungsträger über einen größeren Bereich, so dass dort stärkere Beschleunigungsspannungen und/oder größere Ladungsträgermengen benötigt werden. Dementsprechend ist in deren Nähe eine stärkere Abschirmung vorteilhaft.

Um die Außenbeaufschlagungseinrichtung dennoch möglichst platzsparend ausführen zu können und gleichzeitig dennoch den gesamten benötigeten Oberflächenbereich des Behältnisses mit Ladungsträgern beaufschlagen zu können, weist bei einer bevorzugten Ausführungsform der Vorrichtung zum Sterilisieren von Behältnissen zumindest die erste Außenbeaufschlagungseinrichtung, bevorzugt auch eine weitere Außenbeaufschlagungseinrichtung jeweils ein im Wesentlichen rechteckiges oder ovales Ladungsträgeraustrittsfenster auf, wobei bevorzugt zumindest eine Längsachse des Ladungsträgeraustrittsfensters gegenüber einer Längsachse des mit Ladungsträgern zu beaufschlagenden Behältnisses geneigt ist. Bevorzugt ist die gesamte Außenbeaufschlagungseinrichtung gegenüber einer Längsachse des mit Ladungsträgern zu beaufschlagenden Behältnisses geneigt. Bevorzugt ist das Ladungsträgeraustrittsfenster im Wesentlichen rechteckig.

Als gegenüber der Längsachse des mit Ladungsträgern zu beaufschlagenden Behältnisses geneigt sollen Ladungsträgeraustrittsfenster oder auch Außenbeaufschlagungseinrichtungen verstanden werden, bei denen eine Hauptachse gegenüber der Längsachse des mit Ladungsträgern zu beaufschlagenden Behältnisses geneigt ist. Als Hauptachsen sollen diesbezüglich Achsen verstanden werden, die eine Vorzugsrichtung bzw. maximale Ausdehnung in eine Richtung des Austrittsfensters oder des Außenbeaufschlagungseinrichtungsgehäuses bzw. senkrecht dazu stehende Achsen darstellen. Beispiele dafür sind die große Halbachse und die kleine Halbachse eines ovalen (elliptischen) Austrittsfensters oder Außenbeaufschlagungseinrichtungsgehäuses, die Mittelsenkrechten eines rechteckigen Austrittsfensters oder Außenbeaufschlagungseinrichtungsgehäuses oder andere.

Bevorzugt sind auch die Hauptachsen der Außenbeaufschlagungseinrichtungen gegenüber einander geneigt.

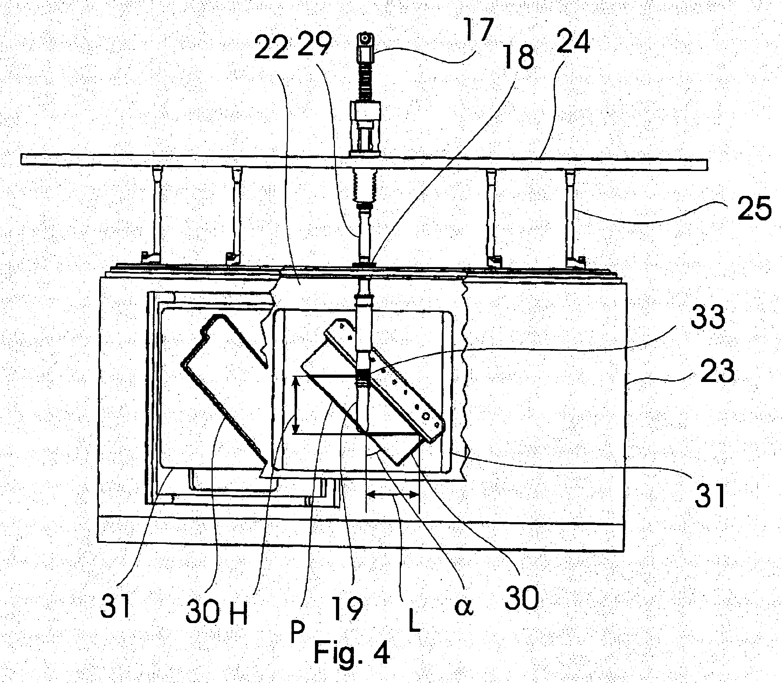

Durch die geneigte Anordnung der Außenbeaufschlagungseinrichtungen und/oder des Ladungsträgeraustrittsfensters kann der Abschnitt in dem ein Behältnis während dessen Transports entlang des Transportpfades sterilisiert wird, verlängert werden. Gleichzeitig kann aber auch gewährleistet werden, dass das Behältnis über dessen gesamte Höhe bzw. Länge jedoch mindestens über den gesamten zu sterilisierenden Bereich der äußeren Oberfläche den Ladungsträgern ausgesetzt ist. Wie detailliert bei der Beschreibung der Fig. 4 dargestellt ist, ergibt sich durch die Neigung des Ladungsträgeraustrittsfensters und/oder der Außenbeaufschlagungseinrichtung ein Bereich in Form eines Parallelogramms, in welchem sich die von dem Behältnis während dessen Transports überstrichene Fläche und die von der Außenbeaufschlagungseinrichtung emittierte Ladungsträgerwolke überschneiden.

Dieser Bereich ist so dimensioniert, dass das Behältnis diesen mit allen zu sterilisierenden Außenoberflächen durchläuft. Bevorzugt weist das diesen Bereich begrenzende Parallelogramm daher eine Höhe (über der Grundlinie) auf, die mindestens der Länge bzw. Höhe des Behältnisses entspricht. Durch die Neigung kann die Länge des Transportpfades, in welchem ein jeweiliger Anteil der äußeren Behältnisoberfläche sterilisiert wird, bevorzugt um ein Verhältnis vergrößert werden, welches (Streueffekte der Ladungsträger unberücksichtigt) ungefähr dem Sinus des Neigungswinkels zur Breite des Ladungsträgeraustrittsfensters entspricht.

Bevorzugt ist weiterhin eine Vorrichtung zum Sterilisieren von Behältnissen bei der die Vorrichtung eine Einführeinrichtung aufweist, mittels welcher die Innenbeaufschlagungseinrichtung zumindest abschnittsweise in das Innere eines Behältnisses einführbar ist, wobei das Behältnis und die zweite Ladungsträgeraustrittseinrichtung, bevorzugt in einer Längsrichtung des Behältnisses, gegenüber einander relativbeweglich sind.

In einer weiteren bevorzugten Ausführungsform der Vorrichtung zum Sterilisieren von Behältnissen kontaktiert ein Abschnitt einer gegenüber dem ersten Anteil der Strahlungsabschirmungsvorrichtung relativbeweglichen Trägerplatte zumindest zeitweise ein in einem Kanal befindliches Dichtungs- und/oder Sterilisationsmedium, um einen sich zumindest abschnittsweise entlang des Transportpfades erstreckenden Reinraum abzudichten. Die Trägerplatte ist bevorzug synchron zu einem gegenüber dem ersten Anteil der Strahlungsabschirmungsvorrichtung relativbeweglichen Anteil der Strahlungsabschirmungsvorrichtung bewegbar. Bevorzugt bildet die Trägerplatte eine Begrenzung des Reinraums.

Der Transportkanal ist demnach auf der Seite des relativbeweglichen Anteils der Strahlungsabschirmungsvorrichtung durch eine hydraulische Dichtung gegenüber der Umgebung abgeschlossen. Bevorzugt handelt es sich um einen Ring, welcher sich entsprechend der Bewegung der Behältnisse entlang des Transportpfades dreht und den Transportpfad, bzw. den Abschnitt des Transportpfades in welchem die Außensterilisation stattfindet abschirmt.

Dadurch kann gewährleistet werden, dass das Innere des Transportkanals gegenüber der Umgebung abgeschlossen ist und keine Fremdkörper, Verunreinigungen, Mikroorganismen, Sporen und andere in den Transportkanal eindringen können. Bevorzugt handelt es sich bei dem Medium um ein Medium, welches sowohl abdichtende als auch sterilisierende Eigenschaften aufweist. Weiter bevorzugt handelt es sich um ein flüssiges oder zähflüssiges (niederviskoses) Medium, so dass im Inneren des Transportkanals ein Überdruck aufgebaut werden kann, der das Einströmen von Umgebungsluft und darin mitgeführten Verunreinigungen in den sterilen Transportkanal verhindert. Es sind jedoch auch Gasse als Medium möglich, wobei diese bevorzugt derart in Strömung versetzt, bzw. gehalten werden, dass ein Eindringen von Fremdstoffen in den Reinraum verhindert wird. Insbesondere wird demnach eine Strömung aufrecht erhalten, welche aus Richtung des Reinraums in Richtung der Umgebung verläuft.

Bevorzugt ist unterhalb des Kanals, in welchem sich das Dichtungs- und/oder Sterilisationsmedium befindet ein weiterer Kanal angeordnet, in welchen Medium aus dem oberen Kanal eingebracht, z.B. eingesaugt, werden kann. Bei dem Dichtungs- und/oder Sterilisationsmedium handelt es sich bevorzugt um eine wässrige Lösung eines sterilisierenden Wirkstoffs (Sterilisationsmittels). Besonders bevorzugt wird als Dichtungsmittel ein Gasstrom verwendet, bevorzugt ein Luftstrom, insbesondere (gefilterte) Umgebungsluft, welcher durch seine Strömung das Eindringen von Verunreinigungen in das Innere des Reinraums verhindert.

Bevorzugt ist weiterhin eine Vorrichtung zum Sterilisieren von Behältnissen bei der der Abschnitt der gegenüber dem ersten Anteil der Strahlungsabschirmungsvorrichtung relativbeweglichen Trägerplatte zumindest zeitweise von dem in dem Kanal befindlichen Dichtungs- und/oder Sterilisationsmedium trennbar ist, um, insbesondere zur Wartung und/oder Reinigung, einen Zugang zu dem sich entlang des Transportpfades erstreckenden Reinraum zu ermöglichen. Insbesondere ist vorgesehen, dass der gegenüber dem ersten Anteil relativbewegliche Anteil der Strahlungsabschirmungsvorrichtung, insbesondere ein Oberteil der Außensterilisationseinrichtung, nach oben abnehmbar ist. Dadurch ergibt sich die Möglichkeit, den zuvor gegenüber der Umgebung abgeschlossenen Transportkanal zu öffnen, um diesen zu reinigen. Beispielsweise kann so sehr einfach ein zusätzliches Desinfektions- bzw. Sterilisationsmittel in den Transportkanal eingeführt werden. Außerdem ist es möglich, evtl. in dem Transportkanal befindliche Behältnisse, welche sich von den Halteeinrichtungen gelöst haben, zu entfernen, um Beschädigung oder Verlust weiterer Behältnisse zu vermeiden.

Weiterhin ist eine Ausführungsform der Vorrichtung zum Sterilisieren von Behältnissen bevorzugt bei der die Vorrichtung eine Hubvorrichtung aufweist, mittels welcher die Behältnisse während deren Transport entlang des Transportpfades entlang deren Längsrichtung bewegbar sind. Bevorzugt befindet sich eine derartige Hubvorrichtung im Bereich einer Transporteinrichtung, welche die Behältnisse während deren Außensterilisation transportiert. Dadurch ist es möglich, die Behältnisse insbesondere während der Außensterilisation gegenüber der Außenbeaufschlagungseinrichtung auszurichten. Insbesondere ist es bei gegenüber der Behältnislängsachse geneigten Ladungsträgeraustrittsfenstern dadurch möglich, die Behältnisse jeweils so zu positionieren, dass der vorgesehene Bereich der äußeren Behältnisoberfläche mit Ladungsträgern beaufschlagt wird.

Bevorzugt weist dieser Hubmechanismus einen Motor und eine Gewindestangenführung auf, die dem geringen im Bereich der Außensterilisationseinrichtun zur Verfügung stehenden Platz angepasst sind. Die besagte Bewegung der Behältnisse in deren Längsrichtung wäre jedoch auch durch die Verwendung von Führungsschienen bzw. Steuerkurven realisierbar.

Bei den Ladungsträgern handelt es sich insbesondere um Elektronen, es wäre jedoch auch denkbar, dass andere Ladungsträger, wie Ionen, verwendet werden.

Das Ladungsträgeraustrittsfenster ist besonders bevorzugt aus einem Material hergestellt, welches aus einer Gruppe von Materialien ausgewählt ist, welche Titan, Quarzglas, Diamant, Kombinationen hieraus und dergleichen enthält.

Weiterhin ist eine Anlage zum Behandeln von Vorformlingen Gegenstand der vorliegenden Erfindung, welche mindestens eine Vorrichtung der oben beschriebenen Art umfasst, wobei diese Vorrichtung bevorzugt stromabwärts bezüglich einer Heizeinrichtung zum Erwärmen von Kunststoffvorformlingen und stromaufwärts einer Abfülleinrichtung, bevorzugt stromaufwärts einer Umformungseinrichtung für Vorformlinge angeordnet ist.

Durch eine derartige Anlage ist es möglich, eine Innen- und Außensterilisierung von Behältnissen insbesondere in den zur Produktion und Befüllung von Behältnissen verwendeten hohen Taktraten und Durchsatzzahlen durchzuführen.

Insbesondere wenn die beschriebene Vorrichtung stromabwärts bezüglich einer Heizeinrichtung und stromaufwärts bezüglich einer Umformungseinrichtung angeordnet ist, ist eine schnelle Sterilisation und ein schneller Transport durch die Vorrichtung notwendig. Dauert der Sterilisationsprozess bzw. der Transport durch die Sterilisationsvorrichtung zu lange, besteht die Gefahr des Abkühlens der Vorformlinge. Bevorzugt ist die Vorrichtung daher geeignet, die Behältnisse innerhalb eines Zeitfensters von weniger als 20 Sekunden, bevorzugt weniger als 15 Sekunden, besonders bevorzugt etwa 11 Sekunden entlang des Transportpfades zu transportieren und dabei zu sterilisieren.

Bevorzugt weist die Anlage eine Transporteinrichtung auf, welche die Behältnisse entlang eines vorgegebenen Transportpfades insbesondere auch während deren Sterilisation bewegt. Vorteilhaft handelt es sich bei der Transporteinrichtung um einen drehbaren Träger, an dem besonders bevorzugt eine Vielzahl von Greifelementen angeordnet ist.

Vorzugsweise weist die Anlage eine Einrichtung zum Befüllen von Behältnissen auf und die erfindungsgemäße Vorrichtung ist stromaufwärts bezüglich dieser Einrichtung angeordnet.

Weiterhin ist bevorzugt, dass die Anlage mindestens ein Transportelement, bevorzugt einen Transportstern aufweist, welcher dazu geeignet ist, ein Behältnis von einer Vorrichtung zum Sterilisieren von Behältnissen zu übernehmen und an eine Vorrichtung zum Umformen der Behältnisse zu übergeben.

Bevorzugt weist eine solche Anlage jeweils einen sogenannten Schleusenstern zum Einführen bzw. zum Ausführen der Behältnisse in bzw. aus einer Vorrichtung der oben beschriebenen Art auf. Bevorzugt unterscheiden sich diese beiden Schleusensterne. Insbesondere aufgrund des innerhalb der Vorrichtung veränderten Teilungsabstands ist eine identische Ausführung der Schleusensterne nicht möglich.

Ein weiterer wesentlicher Aspekt der Erfindung ist ein Verfahren zum Sterilisieren zumindest eines Abschnitts einer Innenwandung und eines Abschnitts einer Außenwandung von Vorformlingen mittels beschleunigter Ladungsträger, wobei der Vorformling durch mindestens eine erste Außenbeaufschlagungseinrichtung zum Sterilisieren zumindest eines Abschnitts einer Außenwandung von Vorformlingen und eine Innenbeaufschlagungseinrichtung zum Sterilisieren zumindest eines Abschnitts einer Innenwandung von Vorformlingen mit Ladungsträgern beaufschlagt wird, wobei die Innenbeaufschlagungseinrichtung zumindest abschnittsweise durch eine Öffnung in den Vorformling eingeführt wird, um eine Innenwandung des Vorformlings mit austretenden Ladungsträgern zu beaufschlagen, wobei die Vorformlinge während deren Sterilisation mit einer Transporteinrichtung entlang eines Transportpfades transportiert werden, wobei entlang eines Abschnitts des Transportpfades der Vorformlinge zwischen der ersten Außenbeaufschlagungseinrichtung und der Innenbeaufschlagungseinrichtung eine Abstandsänderungseinrichtung, angeordnet ist, welche einen Abstands zwischen zwei entlang des Transportpfades aufeinander folgenden Vorformlingen verändert.

Durch dieses Verfahren ist es somit möglich, Vorformlinge in hohen Taktraten sowohl auf deren Außenseite als auch auf deren Innenseite zumindest abschnittsweise zu sterilisieren. Die zwischen der Außenbeaufschlagungseinrichtung und der Innenbeaufschlagungseinrichtung angeordnete Abstandsänderungseinrichtung ermöglicht es, sowohl an der Außenbeaufschlagungseinrichtung als auch an der Innenbeaufschlagungseinrichtung den für den jeweiligen Sterilisationsprozess erforderlichen Abstand und damit auch die Transportgeschwindigkeit der Behältnisse einzustellen.

Bevorzugt ist eine Variante des Verfahrens zum Sterilisieren bei der Vorformlinge, insbesondere Vorformlinge für Getränkebehältnisse und/oder andere fluide Medien, sterilisiert werden. Dies hat den Vorteil, dass im Vergleich zu fertig ausgeformten Behältnissen wesentlich kleinere Oberflächen zu sterilisieren sind und der Prozess somit deutlich effizienter gestaltet werden kann. Außerdem kann die gesamte Vorrichtung kleiner dimensioniert sein, was in einer deutlichen Gewichtsreduzierung resultiert. Weiterhin wird dadurch eine höhere Prozessstabilität der Ladungsträgerbeaufschlagungseinrichtungen, also der Außenbeaufschlagungseinrichtung und der Innenbeaufschlagungseinrichtung, ermöglicht, da die eingesetzte Energie und die resultierende Fensterbelastung aufgrund der Abmessungen eines Vorformlings geringer sind.

Weiterhin bevorzugt ist eine Variante des Verfahrens zum Sterilisieren von Behältnissen bei dem das Behältnis entlang eines Transportpfads transportiert wird, welcher zumindest in einem Abschnitt, in welchem die Außenbeaufschlagungseinrichtung zum Sterilisieren zumindest eines Abschnitts einer Außenwandung von Behältnissen angeordnet ist, gekrümmt ist.

Außerdem ist eine Variante des Verfahrens zum Sterilisieren von Behältnissen bevorzugt auf bei welchem auf der der ersten Außenbeaufschlagungseinrichtung gegenüberliegenden Seite des Transportpfades, eine weitere Außenbeaufschlagungseinrichtung angeordnet ist, wobei die erste Außenbeaufschlagungseinrichtung und die Außenbeaufschlagungseinrichtung bevorzugt entlang des Transportpfades versetzt angeordnet sind. Bevorzugt sind die beiden Außenbeaufschlagungseinrichtungen um einen Winkel zwischen 2 und 20°, besonders bevorzugt zwischen 5 und 10°, eines Krümmungskreises des Transportpfads, gegenüber einander versetzt.

Weitere Vorteile und Ausführungsformen ergeben sich aus den beigefügten Zeichnungen:

Darin zeigen:
- Fig. 1: eine schematische Darstellung einer Aufsicht auf eine Vorrichtung zum Sterilisieren von Vorformlingen;
- Fig. 2: eine schematische Darstellung einer Aufsicht auf einen Bereich einer Vorrichtung zum Sterilisieren von Vorformlingen in welchem die Außensterilisation der Vorformlinge stattfindet;
- Fig. 3: eine schematische Darstellung einer Schrägansicht auf einen Bereich einer Vorrichtung zum Sterilisieren von Vorformlingen in welchem die Außensterilisation der Vorformlinge stattfindet mit zusätzlicher Darstellung eines Ladungsträgeraustrittsfensters;
- Fig. 4: eine schematische Darstellung einer Seitenansicht auf einen Bereich einer Vorrichtung zum Sterilisieren von Vorformlingen in weichem die Außensterilisation der Vorformlinge stattfindet mit zusätzlicher Darstellung der Ladungsträgeraustrittsfenster;
- Fig. 5: eine schematische Darstellung einer Schrägansicht auf einen Bereich des Transportkanals mit Strahlungsabschirmungseinrichtung;
- Fig. 6: eine schematische Darstellung einer Seitenansicht auf einen Bereich des Transportkanals in einem geschlossenen Zustand;
- Fig. 7: eine schematische Darstellung einer Seitenansicht auf einen Bereich des Transportkanals in einem geöffneten Zustand.

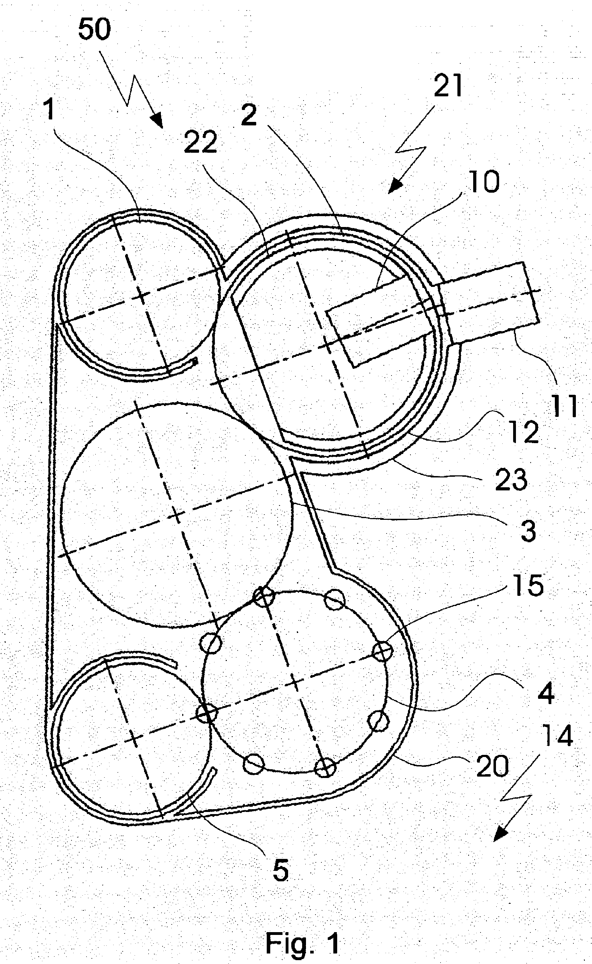

Figur 1 zeigt eine schematische Darstellung einer Aufsicht auf eine Vorrichtung 50 zum Sterilisieren von Behältnissen 19. Diese weist eine Transporteinrichtung 1 zum Aufnehmen von Behältnissen 19 auf, welcher in der gezeigten Ausführungsform als Einlaufstern 1 ausgeführt ist. Stromabwärts bezüglich des Einlaufsterns 1 befindet sich eine Behandlungszone für die Behältnisse 19 in der die Außensterilisation stattfindet. Um die Behältnisse 19 mit Ladungsträgern beaufschlagen zu können, werden die Behältnisse an eine Transporteinrichtung 2 übergeben die die Behältnisse an den Außenbeaufschlagungseinrichtungen 10 und 11 vorbei transportiert. Während des Transports werden die Behältnisse 19 mit Ladungsträgern beaufschlagt, welche von den Außenbeaufschlagungseinrichtungen 10 und 11 emittiert werden. Um eine vollumfängliche Sterilisation zu ermöglichen, sind zwei Außenbeaufschlagungseinrichtungen 10 und 11 vorgesehen, wobei eine erste Außenbeaufschlagungseinrichtung 10 auf der radial innenliegenden Seite des Transportpfades angeordnet ist und eine zweite Außenbeaufschlagungseinrichtung 11 auf der radial außenliegenden Seite des Transportpfades. Denkbar wäre aber auch auf eine der beiden Außenbeaufschlagungseinrichtungen zu verzichten und stattdessen durch eine (z.B. vollständige) Rotation des Behältnisses eine vollständige Sterilisation des Behältnisses zu erreichen.

Zur Abschirmung der Umgebung 14 gegenüber der von den Außenbeaufschlagungseinrichtungen 10 und 11 emittierten Strahlung, insbesondere von der nach außen strahlenden Außenbeaufschlagungseinrichtung 10, ist dieser Bereich von einer starken Strahlungsabschirmungsvorrichtung 21 umgeben. Dabei setzt sich die Strahlungsabschirmungsvorrichtung aus mehreren Strahlungsabschirmungseinrichtungen 22 und 23 zusammen, welche den Transportkanal in verschiedene Richtungen abschirmen. In der gezeigten Aufsicht ist eine äußere Strahlungsabschirmungseinrichtung 23 und eine innere Strahlungsabschirmungseinrichtung 22 zu erkennen. Abschirmungen nach unten und oben sind nicht gezeigt.

Durch die Krümmung des Transportpfades und damit auch der Strahlungsabschirmungsvorrichtung 21 im Bereich der Außenbeaufschlagungseinrichtungen 10 und 11 ist es möglich, dass Strahlung zwischen der äußeren Strahlungsabschirmungseinrichtung 23 und der inneren Strahlungsabschirmungseinrichtung 22 reflektiert wird und so der Bereich, in dem die Sterilisation stattfindet größer ist als lediglich der Bereich unmittelbar vor den Außenbeaufschlagungseinrichtungen 10 und 11 bzw. deren Ladungsträgeraustrittsfenster.

Um eine möglichst lange Verweilzeit der Behältnisse in dem Bereich zu ermöglichen, in welchem die Sterilisation deren äußerer Oberfläche stattfindet, ist eine geringe Transportgeschwindigkeit und ein geringer Abstand zwischen den Behältnissen vorteilhaft. Durch den geringen Abstand zwischen zwei benachbarten Behältnissen entlang des Transportpfades ist auch bei geringer Transportgeschwindigkeit ein hoher Durchsatz zu erzielen. Weiterhin ist bei einem geringen Teilungsabstand vorteilhaft, dass wenige Ladungsträger (oder auch Strahlung) für den Sterilisationsprozess ungenutzt zwischen den Behältnissen hindurch passieren können.

Um jedoch einen größeren Teilungsabstand, also einen größeren Abstand zwischen zwei direkt aufeinander folgenden Behältnissen entlang des Transportpfades, wie er bei stromabwärts benötigten Einrichtungen benötigt wird wieder herzustellen, ist stromabwärts direkt nach der Außensterilisationsvorrichtung eine Abstandsänderungseinrichtung 3, zur Veränderung eines Abstands zwischen zwei entlang des Transportpfades aufeinander folgenden Behältnissen angeordnet. Mittels dieser Abstandsänderungseinrichtung 3, in diesem Fall ein Teilungsverzugsstern, ist es möglich, den Abstand der Behältnisse so zu variieren, dass eine Übergabe an eine stromabwärts angeordnete Innensterilisationseinrichtung 15 möglich ist.

Die Innensterilisationseinrichtung weist eine Vielzahl von Innenbeaufschlagungseinrichtungen 15 auf, welche zumindest teilweise in die Behältnisse eingeführt werden können. Diese sind entlang bzw. um einer Transporteinrichtung 4 angeordnet, welche die Behältnisse während der Behandlung einer inneren Oberfläche der Behältnisse entlang des Transportpfades transportiert. Zur Innensterilisation weist jede der Innenbeaufschlagungseinrichtungen 15 einen sogenannten Strahlfinger auf, der so dimensioniert ist, dass er durch eine Öffnung des Behältnisses passt. Der übrige Teil jeder Innenbeaufschlagungseinrichtung 15 ist üblicherweise jedoch deutlich größer als der Strahlfinger und weist insbesondere einen größeren Durchmesser auf. Der Durchmesser ist üblicherweise auch größer als der jedes einzelnen zu sterilisierenden Behältnisses, so dass der Abstand zwischen zwei entlang des Transportpfades direkt aufeinander folgender Behältnisse im Bereich der Innensterilisationseinrichtung nicht mehr durch den Durchmesser der Behältnisse, sondern durch einen minimalen Abstand zwischen zwei benachbarten Innenbeaufschlagungseinrichtungen 15 vorgegeben ist. Insbesondere bei der Innensterilisation von Vorformlingen ist es daher notwendig, den Abstand zwischen zwei entlang des Transportpfades direkt aufeinander folgenden Behältnisse zu vergrößern und auf den Abstand zwischen zwei benachbarten Innenbeaufschlagungseinrichtungen 15 anzupassen.

Von der Innensterilisationseinrichtung werden die Behältnisse nach der Behandlung mit einer Innenbeaufschlagungseinrichtungen 15 an eine stromabwärts angeordnete Transporteinrichtung 5 übergeben. Diese Transporteinrichtung 5 ist wie auch die Transporteinrichtung 1 als Transportstern 5 ausgeführt. Die Transportsterne 1 und 5 unterscheiden sich jedoch in Ihrem Aufbau. Mindestens der Teilungsabstand, mit welchem sie die Behältnisse transportieren, ist unterschiedlich. Der Transportstern 5 übernimmt die auf einer inneren Oberfläche sterilisierten Behältnisse von einer Transporteinrichtung 4, die die Behältnisse während der Innensterilisation transportiert und übergibt sie an eine weitere (nicht gezeigte) Transporteinrichtung oder eine Behältnisbehandlungseinrichtung. Beispielsweise könnte es sich bei einer stromabwärts des Transportsterns 5 angeordneten Behältnisbehandlungseinrichtung um eine Umformungseinrichtung oder um eine Abfülleinrichtung handeln. Die einzelnen Innenbeaufschlagungseinrichtungen 15 beaufschlagen vorteilhaft die gesamte Innenoberfläche der Behältnisse 19 mit Ladungsträgern.

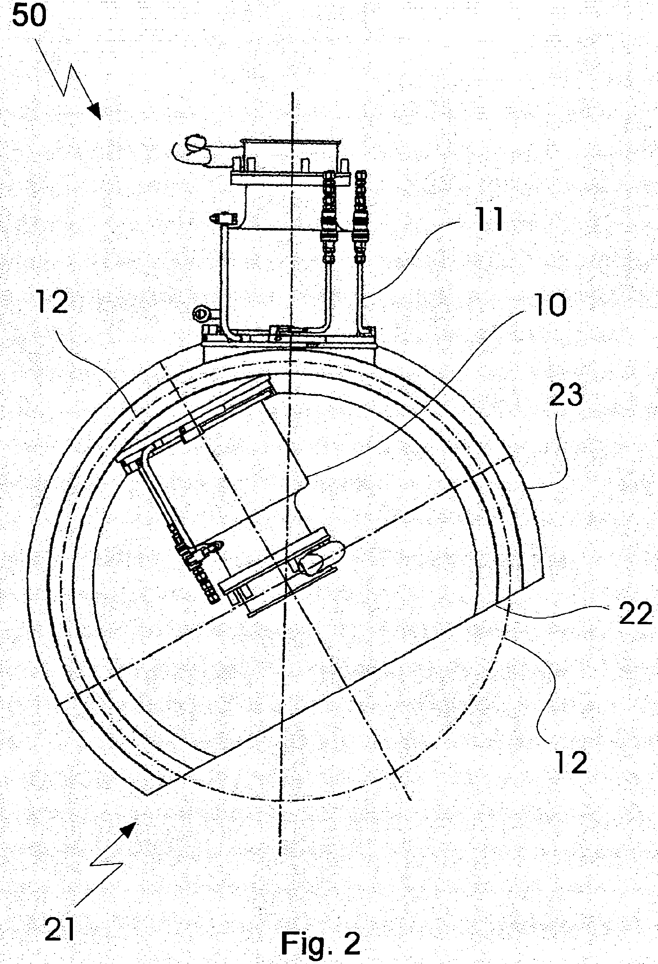

In Figur 2 ist eine schematische Darstellung einer Aufsicht auf einen Bereich einer Vorrichtung 50 zum Sterilisieren von Behältnissen 19 in welchem die Außensterilisation der Behältnisse 19 stattfindet gezeigt.

Insbesondere zeigt Fig. 2 einen Abschnitt des Transportpfades, entlang welchem die Außensterilisation der Behältnisse 19 stattfindet. Zur Außensterilisation werden die Behältnisse an den Außenbeaufschlagungseinrichtungen 10 und 11 vorbeigeführt. Diese Außenbeaufschlagungseinrichtungen 10 und 11 sind auf verschiedenen Seiten des Transportpfades angeordnet und sind somit dazu geeignet, ein Behältnis 19 von verschiedenen Seiten mit Ladungsträgern zu beaufschlagen. Dadurch kann der Transportmechanismus für die Behältnisse 19 vereinfacht werden, da eine vollständige Drehung der Behältnisse 19 zur vollumfänglichen Ladungsträgerbeaufschlagung nicht notwendig ist. Vorteilhaft beaufschlagen die Außenbeaufschlagungseinrichtungen 10 und 11 die gesamte äußere Umfangsfläche der Behältnisse 19 mit Ladungsträgern.

Die beiden Außenbeaufschlagungseinrichtungen 10 und 11 stehen sich nicht direkt gegenüber, sondern sind gegenüber einander entlang des Transportpfades der Behältnisse 19 versetzt angeordnet. Bei einem Transport der Behältnisse entlang des Transportpfades, welcher bei der in Fig. 2 gezeigten Aufsicht im Uhrzeigersinn (rechtsdrehend) verläuft, gelangen die Behältnisse somit zunächst in den Einflussbereich der radial innenliegenden und Ladungsträger radial nach außen beschleunigenden Außenbeaufschlagungseinrichtung 10. Dort wird eine bezüglich der Transporteinrichtung 2 radial innenliegenden Außenoberfläche des Behältnisses mit Ladungsträgern beaufschlagt.

Erst etwas weiter stromabwärts gelangen die Behältnisse 19 in den Einflussbereich der radial außenliegenden und Ladungsträger radial nach innen beschleunigenden Außenbeaufschlagungseinrichtung 11. Die bei der Behandlung mit der anderen Außenbeaufschlagungseinrichtung 10 im Ladungsträgerschatten des Behältnisses liegende, bezüglich der Transporteinrichtung 2 radial außenliegende, Außenoberfläche des Behältnisses wird in diesem Bereich durch die Außenbeaufschlagungseinrichtung 11 mit Ladungsträgern beaufschlagt.

Die beiden Außenbeaufschlagungseinrichtungen 10 und 11 sind bezüglich des Zentrums bzw. der Rotationsachse der Transporteinrichtung 2 um einen vorgegebenen Winkel versetzt angeordnet. Durch diesen Versatz kann gewährleistet werden, dass die Ladungsträger beider Außenbeaufschlagungseinrichtungen 10 und 11 nicht direkt aufeinander zu beschleunigt werden, wodurch im Dauerbetrieb evtl. Beschädigungen der Außenbeaufschlagungseinrichtungen 10 und 11 hervorgerufen werden könnten. Außerdem können die Behältnisse 19 bei der Beaufschlagung mit den Ladungsträgern erwärmt werden, indem sie die kinetische Energie der beschleunigten Ladungsträger zumindest teilweise absorbieren und in thermische Energie umwandeln. Durch die versetzte Anordnung der Außenbeaufschlagungseinrichtungen 10 und 11 kann somit eine Überhitzung und damit eine mögliche Beschädigung der Behältnisse bei gleichzeitiger Ladungsträgerbeaufschlagung aus zwei Außenbeaufschlagungseinrichtungen 10 und 11 vermieden werden.

Darüber hinaus wird die Länge des Abschnitts des Transportpfades, in welchem die Behältnisse mit Ladungsträgern beaufschlagt werden, verlängert. Bei einer wolkenartigen Ausbreitung der Ladungsträger, bei welcher die Ladungsträger zusätzlich zu durch die Ladungsträgerbeschleunigungseinrichtung vorgegebenen Vorzugsrichtung auch einer Diffusion unterliegen, ist somit auch eine Überlappung der jeweils emittierten Ladungsträgerwolken möglich, so dass sich die Behältnisse 19 insgesamt länger in der gemeinsamen Ladungsträgerwolke beider Außenbeaufschlagungseinrichtungen 10 und 11 befinden.

Zur Abschirmung der Umgebung 14 gegenüber den emittierten Ladungsträgern und/oder bei der Ladungsträgererzeugung entstehender Strahlung ist der Bereich der Außensterilisation mit einer starken Strahlungsabschirmungsvorrichtung 21 umgeben. Diese besteht mindestens aus den Strahlungsabschirmungseinrichtungen 22 und 23, welche den Transportpfad auf verschiedenen Seiten umgeben. Bezüglich des Zentrums der Transporteinrichtung 2 (insbesondere einer Drehachse der Transporteinrichtung 2) ist eine Strahlungsabschirmungseinrichtung 23 radial außerhalb des Transportkanals angeordnet und eine Strahlungsabschirmungseinrichtung 22 radial innerhalb des Transportkanals angeordnet. Abschirmungen des Transportkanals senkrecht zur Zeichnungsebene sind nicht gezeigt.

Die Strahlungsabschirmungsvorrichtung 21 folgt im Bereich der Außenbeaufschlagungseinrichtungen 10 und 11 der Krümmung des Transportpfades und ermöglicht somit, dass Strahlung zwischen der äußeren Strahlungsabschirmungseinrichtung 23 und der inneren Strahlungsabschirmungseinrichtung 22 reflektiert wird. Dadurch kann der Bereich, in welchem die Behältnisse Ladungsträgern und/oder energiereicher (sterilisierender) Strahlung ausgesetzt sind, vergrößert werden.

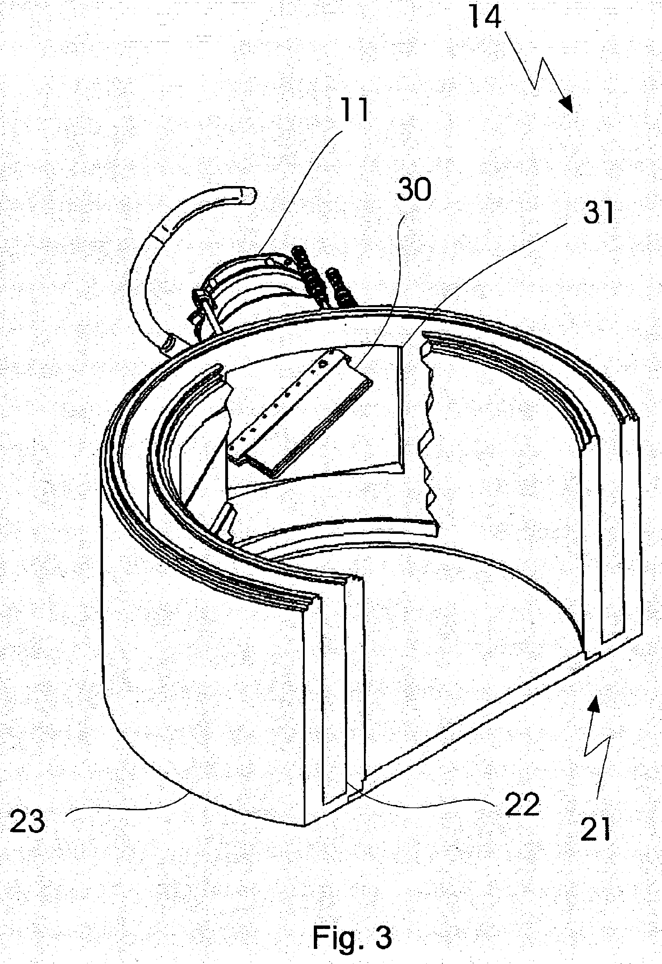

Fig. 3 zeigt eine schematische Darstellung einer Schrägansicht auf einen Bereich einer Vorrichtung zum Sterilisieren von Behältnissen in welchem die Außensterilisation der Behältnisse 19 stattfindet mit zusätzlicher Darstellung eines Ladungsträgeraustrittsfensters 30.

Wie auch in Fig. 2 sind der von der Strahlungsabschirmungsvorrichtung 21 umgebene Transportkanal und eine Außenbeaufschlagungseinrichtung 11 gezeigt. Die bezüglich des Zentrums der (nicht gezeigten) Transporteinrichtung 2 radial innenliegende Außenbeaufschlagungseinrichtung 10 sowie der in diesem Bereich befindliche Teil der inneren Strahlungsabschirmungseinrichtung 22 sind nicht gezeigt, um die Anordnung der Außenbeaufschlagungseinrichtung 11 und deren Ladungsträgeraustrittsfenster 30 darstellen zu können.

Wie in Fig. 3 zu erkennen ist, ist die äußere Strahlungsabschirmungseinrichtung 23 im Bereich der Außenbeaufschlagungseinrichtung 11 unterbrochen, um den in der Außenbeaufschlagungseinrichtung 11 erzeugten Ladungsträgern einen Eintritt in den Transportkanal zu ermöglichen. Dazu schließt ein Gehäuse der Außenbeaufschlagungseinrichtung 11 den Transportkanal bündig ab, um Kontaminationen des als Reinraum ausgeführten Transportkanals zu verhindern. Realisert wird der bündige Anschluss der Außenbeaufschlagungseinrichtung 11 durch ein Befestigungselement 31, in diesem Fall einen Befestigungsflansch. Um einen ungehinderten Durchgang der Ladungsträger in den Reinraum bzw. Transportkanal zu ermöglichen, weist die Außenbeaufschlagungseinrichtung 11 ein Ladungsträgeraustrittsfenster 30 auf, durch welches Ladungsträger von einer Ladungsträgererzeugungseinrichtung hindurch in Richtung des Transportkanals beschleunigt werden können. Somit ist es möglich, dass die Ladungsträger durch das Ladungsträgeraustrittsfenster 30 in das Innere des Reinraums gelangen können und auf ein dort entlang des Transportpfades transportiertes Behältnis 19 treffen können.

Das Ladungsträgeraustrittsfenster 30 weist einen im Wesentlichen viereckigen Querschnitt auf. Auch wenn andere Geometrien des Ladungsträgeraustrittsfensters 30 wie Kreise, Ovale oder ein Quadrat als Spezialform eines Rechtecks ebenfalls möglich sind, ist eine Rechtecksform oder Parallelogrammform mit ungleichen Seitenlängen bevorzugt. Bei der gezeigten Rechtecksform sind die Hauptachsen gegenüber dem Transportpfad (bzw. dessen horizontalem Vektoranteil) geneigt ausgerichtet. Dadurch ist es möglich, dass sich ein an dem Ladungsträgeraustrittsfenster 30 vorbeigeführtes Behältnis 19 länger im Einflussbereich der Ladungsträgerwolke befindet. Dadurch ist bei vergleichbarer Sterilisationsleistung eine kompaktere Bauweise realisierbar.

Insbesondere dann, wenn durch einen Hubmechanismus das Behältnis 19 während des Transports entlang der Kreisbahn des Transportkanals angehoben werden kann und so dem Verlauf des Ladungsträgeraustrittsfensters 30 folgen kann ist eine Verlängerung des Behandlungsbereichs möglich.

Dies wird insbesondere in der in Fig. 4 gezeigten schematischen Darstellung einer Seitenansicht deutlich. Fig. 4 zeigt eine Seitenansicht eines Bereichs einer Vorrichtung 50 zum Sterilisieren von Behältnissen 19 in welchem die Außensterilisation der Behältnisse stattfindet mit zusätzlicher Darstellung der Ladungsträgeraustrittsfenster 30. Zur Verdeutlichung, dass die Ladungsträgeraustrittsfenster 30 mehrerer Außenbeaufschlagungseinrichtungen 10 und 11 gegenüber dem Transportpfad (bzw. dessen horizontalem Vektoranteil) und/oder der Behältnislängsachse um einen Winkel α geneigt angeordnet sein können, sind mehrere Ladungsträgeraustrittsfenster 30 gezeigt. Diese können auch gegenüber einander geneigt angeordnet sein. Dadurch ist es möglich, dass die im Bereich einer entlang des Transportpfades ersten Außenbeaufschlagungseinrichtung 10 angehobenen (bzw. in eine Richtung entlang der Behältnislängsachse bewegten) Behältnisse 19 im Bereich der entlang des Transportpfades folgenden Außenbeaufschlagungseinrichtung 11 wieder abgesenkt (bzw. in die entlang der Behältnislängsachse der ersten Bewegung entgegengesetzte Richtung bewegt) werden können.

Durch die gegenüber der Behältnislängsachse um den Winkel α geneigte Ausrichtung des Ladungsträgeraustrittsfensters 30 wird die Strecke, entlang welcher bei einem Transport des Behältnisses 19 dessen Außensterilisation stattfinden kann, verlängert. Bereits bei einer ausschließlichen Bewegung des Behältnisses 19 senkrecht zu dessen Längsachse, verlängert sich die Strecke des Transportpfades, entlang welcher ein Abschnitt der Außenwandung der Behältnisse 19 der Ladungsträgerbestrahlung ausgesetzt ist. Betrachtet man beispielsweise die seitliche Ummantelung der Basis eines Behältnisses 19, befindet sich diese entlang eines Abschnitts (Länge L des eingezeichneten Parallelogramms P) des Transportpfades in direktem Einfluss der senkrecht durch die Außenbeaufschlagungseinrichtung 11 auf das Behältnis abgegebener Ladungsträger. Diese Länge L ist (entsprechend den trigonometrischen Funktionen bzw. Winkelfunktion) gegenüber der Breite des Austrittsfensters um einen Faktor verlängert, welcher dem Kehrwert des Sinus des Neigungswinkels (also dem Kosekans des Neigungswinkels) entspricht.

Die Neigung des Ladungsträgeraustrittsfensters 30 ist dabei so gewählt, dass der Überlappungsbereich des Ladungsträgeraustrittsfensters 30 mit der während des Transports des Behältnisses 19 von dessen senkrechter Projektion auf dem Ladungsträgeraustrittsfenster 30 überstrichener Fläche, mindestens eine Höhe H aufweist, die der Länge des Behältnisses in dessen Längsrichtung entspricht. Im gezeigten Beispiel hat der Überlappungsbereich die Form eines Parallelogramms der Länge L und der Höhe H (über L). Je nach Form des Ladungsträgeraustrittsfensters 30, Ausrichtung des Behältnisses 19 bezüglich des Transportpfades und des Verlaufs des Transportpfades sind jedoch auch andere Geometrien des Überlappungsbereichs möglich.

Um eine besonders kompakte Ausführung der Vorrichtung zu ermöglichen, ist wie in Fig. 4 dargestellt vorgesehen, dass das Behältnis 19 während dessen Transports entlang des Transportpfades zusätzlich auch (zumindest mit einer Vektorkomponente) entlang der Behältnislängsachse bewegbar ist. Wird das in Fig. 4 gezeigte Behältnis 19 während dessen Sterilisation nicht nur horizontal (bzw. parallel zur Länge L) bewegt, sondern zusätzlich auch in Höhenrichtung H bewegt, ist es möglich, das Behältnis noch länger im Einflussbereich der Ladungsträger zu halten.

Dazu weist die Transporteinrichtung 2 eine Hubeinrichtung 17 auf. Diese Hubeinrichtung 17 ist mit einer im Wesentlichen horizontalen Trägerplatte 24 verbunden, welche mit der Transporteinrichtung verbunden ist und deren horizontaler Bewegung, in diesem Fall der Rotationsbewegung der Transporteinrichtung 2, folgt. Die Hubeinrichtung 17 ist außerhalb des Reinraums angeordnet. Dadurch werden Wartungsarbeiten vereinfacht. Ein Halteelement 33, welches das Behältnis während dessen Transports entlang des Transportpfades trägt, befindet sich im Inneren des Reinraums und ist mit der Hubeinrichtung 17 verbunden. Die Verbindung zwischen Halteelement 33 und Hubeinrichtung 17 ist mittels eines Trägers 29 realisiert, welcher eine obere Strahlungsabschirmungseinrichtung 24 durchdringt. Die obere Strahlungsabschirmungseinrichtung 16 ist gegenüber der Strahlungsabschirmungseinrichtungen 22 und 23 relativbeweglich gelagert und bewegt sich parallel zu der Trägerplatte 24. Die Strahlungsabschirmungseinrichtung 16 weist daher Öffnungen auf, durch welche der Träger 29 geführt werden kann. Um die abschirmenden Eigenschaften der Strahlungsabschirmungseinrichtung 16 auch bei einer Bewegung des Trägers 29 bzw. des Behältnisses in dessen Längsrichtung aufrecht erhalten zu können, weist der Träger 29 zumindest abschnittsweise ein abschirmendes Material auf, durch welches die Öffnungen in der Strahlungsabschirmungseinrichtung 16 sowohl in Bezug auf Ladungsträger und Strahlungen, als auch in Bezug auf Kontaminationen des Reinraums abgedichtet werden können.

Um zu gewährleisten, dass die mit der Trägerplatte 24 verbundenen Hubeinrichtungen 17 und die Öffnungen in der Strahlungsabschirmungseinrichtung 16 derart aufeinander abgestimmt bewegen, dass eine Hubeinrichtung 17 über einer Öffnungen in der Strahlungsabschirmungseinrichtung 16 angeordnet ist, sind die Trägerplatte 24 und die Strahlungsabschirmungseinrichtung 16 über Trägerelemente 25 miteinander verbunden.

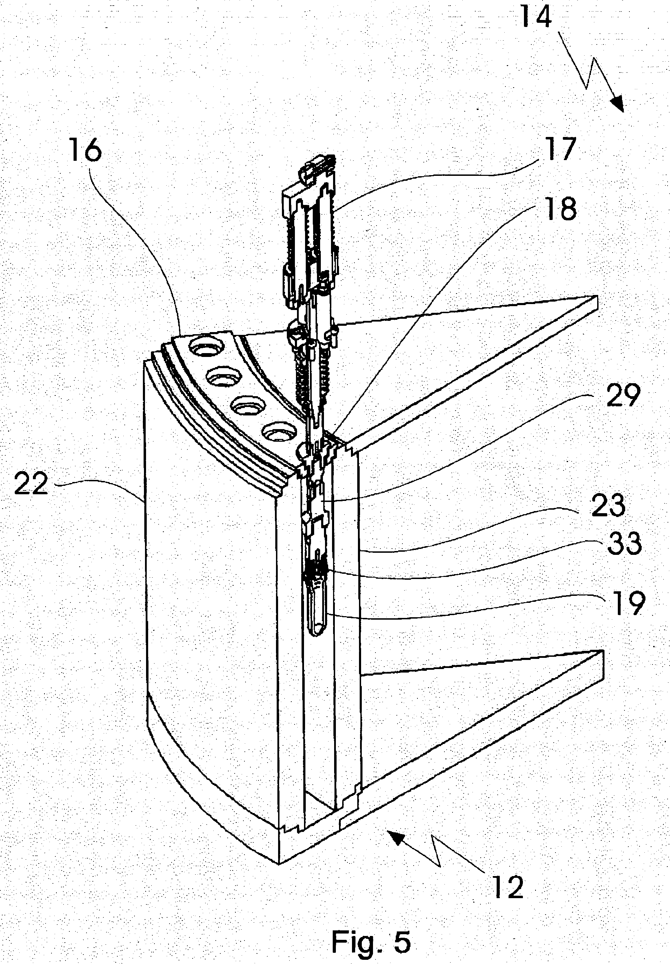

In Fig. 5 zeigt die schematische Darstellung einer Schrägansicht auf einen Bereich des Transportkanals mit Strahlungsabschirmungseinrichtung, wie die Hubbewegung und die Abdichtung des Reinraums während der Hubbewegung erfolgt. Zu erkennen sind die äußere Strahlungsabschirmungseinrichtung 22 und die innere Strahlungsabschirmungseinrichtung 23, zwischen denen sich der Reinraum befindet. Im gezeigten Beispiel sind beide Strahlungsabschirmungseinrichtungen 22 und 23 unterseitig miteinander verbunden, so dass sie gemeinsam im Querschnitt eine U-Form aufweisen. Somit ist auch die Unterseite des Reinraums gegenüber der Umgebung 14 abgedichtet. Sowohl der Eintritt von Verunreinigungen als auch der Austritt von Ladungsträgern und/oder Strahlung wird verhindert. Die Außenbeaufschlagungseinrichtungen 10 und 11 sind zur besseren Übersicht nicht gezeigt.

Da die großen und mit diversen Versorgungsanschlüssen versehenen Außenbeaufschlagungseinrichtungen 10 und 11 möglichst stationär angeordnet sind, sind auch die Strahlungsabschirmungseinrichtungen 22 und 23 gegenüber der Umgebung 14 weitgehend unbeweglich angeordnet. Demgegenüber ist eine obere Strahlungsabschirmungseinrichtung 16 gegenüber den Strahlungsabschirmungseinrichtungen 22 und 23 relativbeweglich angeordnet. Sie bewegt sich entlang einer Kreisbahn. Zur Vermeidung von Kontaminationen des Reinraums ist zwischen den Strahlungsabschirmungseinrichtungen 22 und 23 und der Strahlungsabschirmungseinrichtung 16 eine Dichtung vorgesehen, bevorzugt eine hydraulische Dichtung bzw. ein sogenanntes "Wasserschloss". Das Dichtungsmaterial ist dabei ein Fluid, bevorzugt eine z.B. wässrige Lösung eines Sterilisationsmittels. Die Strahlungsabschirmungseinrichtung 16 weist Öffnungen auf durch welche die Träger 29 hindurchtreten können. Reinraumseitig weisen diese Träger 29 ein Halteelement 33 auf, welches das Behältnis 19 greift. Bevorzugt handelt es sich dabei um einen Dorn, welcher in die Mündung des Behältnisses eingreift. Außerhalb des Reinraums 12 ist der Träger 29 mit einer Hubeinrichtung 17 verbunden, die eine Bewegung des Trägers 29 und somit auch des Behältnisses in deren Längsrichtung (Lᵣ) ermöglicht.

Zur Vermeidung des Austritts von Ladungsträgern und/oder Strahlung aus dem Reinrauminneren in die Umgebung weist der Träger 29 eine separate Strahlungsabschirmungseinrichtung 18 auf, welche die Öffnungen umgebungsseitig und/oder reinraumseitig verschließen kann. Bevorzugt weist der Träger 29 in dem Abschnitt, welcher während einer Hubbewegung die Öffnung in der Strahlungsabschirmungseinrichtung 16 durchdringt einen Außendurchmesser auf, welcher nur geringfügig kleiner ist als ein Innendurchmesser der Öffnung. Bevorzugt ist dieser Bereich durch mindestens eine weitgehend Ladungsträger und/oder Strahlungsundurchlässige Dichtung gegen Ladungsträger- und/oder Strahlungsübertritt in die Umgebung abgesichert. Zur Vermeidung des Einbringens von Kontaminationen aus der Umgebung in das Innere des Reinraums ist bevorzugt reinraumseitig eine Abdichtung mittels eines (nicht gezeigten) Dichtungselements, z.B. einem Faltenbalg 34 vorgesehen.

Allgemein wird daher vorgeschlagen, dass mindestens eine Strahlungsabschirmungseinrichtung 16, 22, 23 auch wenigstens teilweise die Reinraumgrenzen ausbildet.

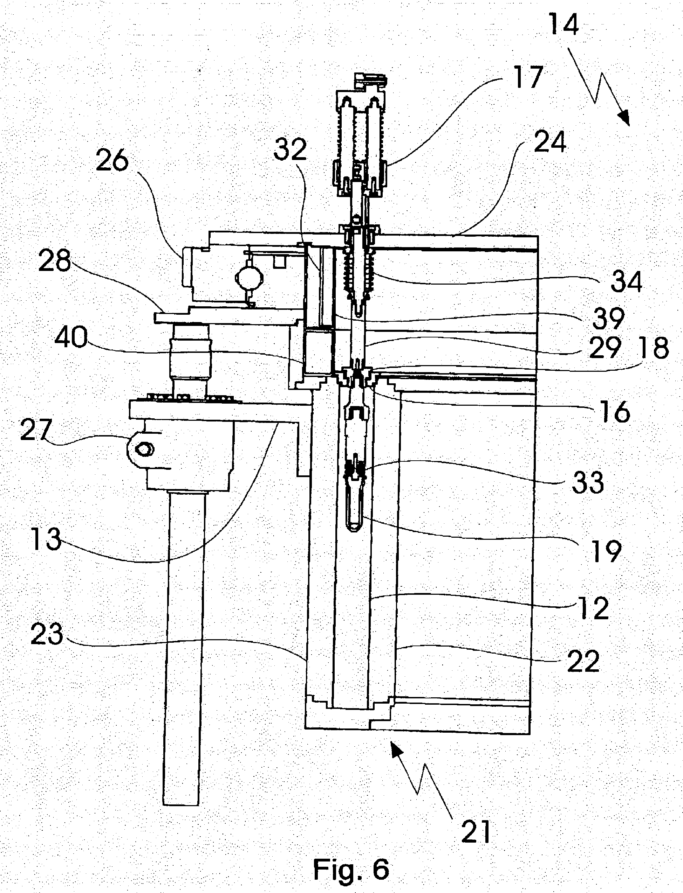

Fig. 6 zeigt eine schematische Darstellung einer Seitenansicht auf einen Bereich des Transportkanals 12 in einem geschlossenen Zustand. Die Abschirmung (gegen Strahlung und/oder Ladungsträgern) und die Abdichtung (gegen Kontaminationen des Reinraums) wird aus dieser Fig. besonders gut ersichtlich.

In dem gezeigten Beispiel ist, wie auch in Fig. 5, der Reinraum 12 mit der ihn umgebenden Strahlungsabschirmungsvorrichtung 21 mit den Strahlungsabschirmungseinrichtungen 22 und 23 gezeigt. Im Inneren des Reinraums 12 befindet sich ein Behältnis 19, welches durch ein Halteelement 33 getragen wird. Das Halteelement 33 steht mit einem Träger 29 in Verbindung, welcher eine Öffnung in der Strahlungsabschirmungseinrichtung 16 durchdringt. Durch Verdickungen (Strahlungsabschirmungseinrichtung 18) des Trägers 29 wird ein sicherer Verschluss der Öffnungen und ein Austreten von Ladungsträgern und/oder Strahlung vermieden. Da sich im gezeigten Beispiel der Reinraum auch oberhalb der Strahlungsabschirmungseinrichtung 16 fortsetzt, ist eine Dichtung zu Verhinderung von Kontaminationen im Bereich der Öffnungen in der Strahlungsabschirmungseinrichtung 16 nicht notwendig. Gegenüber den stationären Strahlungsabschirmungseinrichtungen 22 und 23 ist der Reinraum im Übergangsbereich zur dazu relativbeweglichen Strahlungsabschirmungseinrichtung 16 jedoch durch eine hydraulische Dichtung abgedichtet. Oberseitig wird der Reinraum 12 durch die Trägerplatte 24 begrenzt, an welcher die Hubeinrichtung 17 angeordnet ist. Der Träger 29 durchdringt auch die Trägerplatte 24. In diesem Übergangsbereich ist mindestens ein Anteil des Trägers, welcher sowohl innerhalb als auch außerhalb des Reinraums angeordnet sein kann, mittels eines Faltenbalgs 34 abgedichtet. So kann das Einbringen von Kontaminationen beim Wiedereintreten des Trägers 29 in den Reinraum 12 vermieden werden.

Bezugszeichen 32 bezeichnet ein Grenzelement, welches eine Grenze zwischen sterilem und nicht sterilem Bereich der Vorrichtung 50 darstellt. Dieses Grenzelement 32 taucht in ein Fluid ein, welches in dem Fluidkanal 39 geführt wird. Bevorzugt handelt es sich bei dem Fluid um ein wässriges Sterilisationsmittel, jedoch sind auch andere Fluide bzw. Gase möglich. Insbesondere ist eine Ausführungsform vorgesehen, in welcher die Abdichtung des Reinraums 12 im Bereich der gegenüber einander relativbeweglichen Strahlungsabschirmungseinrichtungen 16, 22 und 23 durch eine spezielle Luftführung realisiert wird. Dazu ist der zusätzliche Kanal 40 vorgesehen, durch welchen kontinuierlich Luft abgesaugt wird. Durch den im Inneren des Reinraums 12 aufrechterhaltenen leichten Überdruck kann so ein kontinuierlicher Gasstrom aus dem Reinraum 12 hinaus aufrecht erhalten werden, welcher das Einströmen von Verunreinigungen verhindert.

Zum Öffnen des Reinraums 12 ist ein weiterer Hubmechanismus 27 vorgesehen, welcher die Trägerplatte 24 einschließlich aller darauf angeordneten Hubeinrichtungen 17, Träger 29, Halteelemente 33 und Behältnisse 19 und die obere Strahlungsabschirmungseinrichtung 16 anhebt. Dazu weist der Hubmechanismus 27 eine weitere Trägerplatte 28 auf, mit welcher die Trägerplatte 24 über einen Kugeldrehverbinder 26 verbunden ist. Dadurch kann der Hubmechanismus 27 stationär angeordnet sein und muss nicht der (Dreh-) Bewegung der Trägerplatte 24 und der Transporteinrichtung 2 folgen. Der Hubmechanismus 27 kann daher über ein Verbindungselement 13 mit der Strahlungsabschirmungseinrichtung 23 verbunden sein.

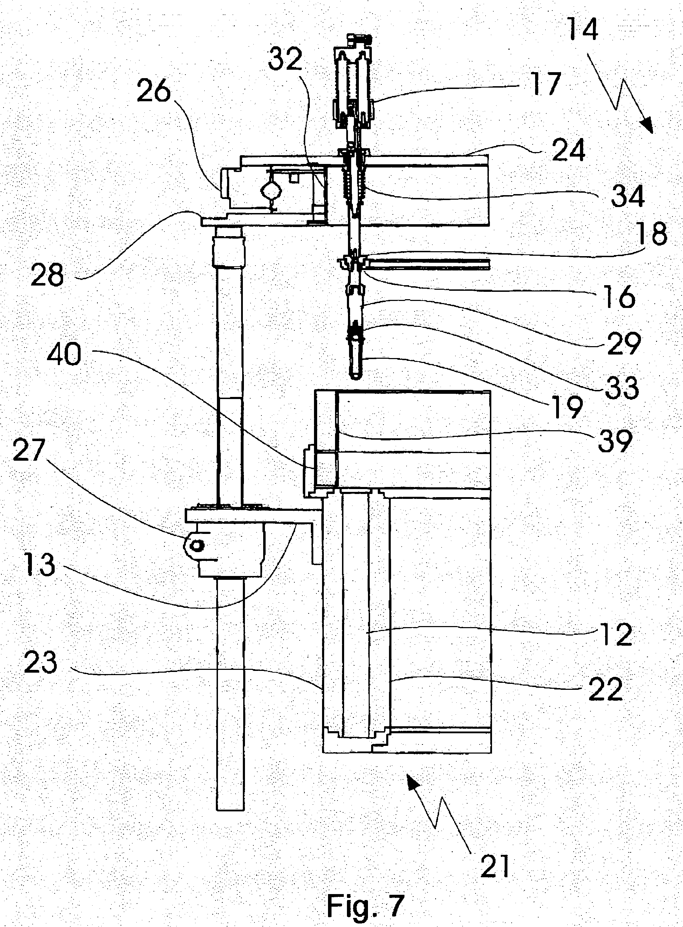

Wie ein Bereich des Transportkanals in einem geöffneten Zustand aussieht, ist schematisch in der in Fig. 7 gezeigten Seitenansicht dargestellt. Durch den nun ausgefahrenen Hubmechanismus 27 ist die Trägerplatte 24 einschließlich der darauf angeordneten Hubeinrichtungen 17, Träger 29, Halteelemente 33 und Behältnisse 19 sowie die obere Strahlungsabschirmungseinrichtung 16 von der Strahlungsabschirmungsvorrichtung 21 abgehoben worden. Gleichzeitig wurde auch das Grenzelement 32 aus dem Kanal 39 herausgezogen worden, so dass der Reinraum 12 nicht mehr gegenüber der Umgebung 14 abgegrenzt ist. Durch das Anheben der Strahlungsabschirmungseinrichtung 16 ist auch die Abschirmung der Umgebung gegen Ladungsträger und/oder Strahlung nicht mehr gewährleistet.

Durch das Anheben großer Teile der Transporteinrichtung 2 mittels des Hubmechanismus 27 ist es sehr einfach möglich, das Innere des Reinraums 12 zugänglich zu machen. Dies kann beispielsweise notwendig sein, wenn sich Verunreinigungen im Reinraum angesammelt haben. Dies können beispielsweise Kontaminationen sein, oder auch Behältnisse 19, die sich während des Transports von den Haltelementent 33 gelöst haben. Durch die Abdichtung der anhebbaren Teile gegenüber den stationären Teilen der Transporteinrichtung mittels einer hydraulischen Dichtung ist es sehr einfach möglich, den Reinraum wieder zu verschließen. Nach dem Absenken der anhebbaren Teile mittels des Hubmechanismus 27 ist nach dem Eintauchen des Grenzelements 32 in das im Kanal 39 geführte Fluid die sterile Abdichtung des Reinraums gegenüber der Umgebung wieder hergestellt.

### Bezugszeichenliste

- 1: Transporteinrichtung, Einlaufstern
- 2: Transporteinrichtung Außensterilisation
- 3: Abstandsänderungseinrichtung
- 4: Transporteinrichtung Innensterilisation
- 5: Transporteinrichtung, Auslaufstern
- 10: Außenbeaufschlagungseinrichtungen
- 11: Außenbeaufschlagungseinrichtungen
- 12: Reinraum
- 13: Verbindungselement
- 14: Umgebung
- 15: Innenbeaufschlagungseinrichtungen
- 16: Obere Strahlungsabschirmungseinrichtung
- 17: Hubeinrichtung
- 18: Strahlungsabschirmungseinrichtung des Trägers
- 19: Behältnis/erfindungsgemäß Vorformling
- 20: Strahlungsabschirmungseinrichtung Innensterilisation
- 21: Strahlungsabschirmungsvorrichtung
- 22: Radial innere Strahlungsabschirmungseinrichtung
- 23: Radial äußere Strahlungsabschirmungseinrichtung
- 24: Erste Trägerplatte
- 25: Trägerelement
- 26: Kugeldrehverbinder
- 27: Hubmechanismus
- 28: Zweite Trägerplatte
- 29: Träger
- 30: Ladungsträgeraustrittsfenster
- 31: Befestigungselement
- 32: Grenzelement
- 33: Halteelement
- 34: Faltenbalg
- 39: Erster Kanal
- 40: Zweiter Kanal
- 50: Vorrichtung zum Sterilisieren
- T: Transportrichtung
- H: Höhe
- L: Länge
- α: Winkel
- P: Parallelogramm

## Patentansprüche

1. Vorrichtung (50) zum Sterilisieren zumindest eines Abschnitts einer Innenwandung und eines Abschnitts einer Außenwandung von Vorformlingen (19) mittels beschleunigter Ladungsträger mit mindestens einer ersten Außenbeaufschlagungseinrichtung (10) zum Sterilisieren zumindest eines Abschnitts einer Außenwandung von Vorformlingen (19) und einer Innenbeaufschlagungseinrichtung (15) zum Sterilisieren zumindest eines Abschnitts einer Innenwandung von Vorformlingen (19), wobei die Innenbeaufschlagungseinrichtung (15) zumindest abschnittsweise durch eine Öffnung in den Vorformling (19) einführbar ist, um eine Innenwandung des Vorformlings (19) mit austretenden Ladungsträgern zu beaufschlagen, mit einer, Transporteinrichtung (2, 4), mittels welcher die Vorformlinge (19) während deren Sterilisation entlang eines Transportpfades transportierbar sind,
**dadurch gekennzeichnet, dass**
entlang eines Abschnitts des Transportpfades der Vorformlinge (19) zwischen der ersten Außenbeaufschlagungseinrichtung (10) und der Innenbeaufschlagungseinrichtung (15) eine Abstandsänderungseinrichtung (3), zur Veränderung eines Abstands zwischen zwei entlang des Transportpfades aufeinander folgenden Vorformlingen (19) angeordnet ist.

2. Vorrichtung (50) zum Sterilisieren von Vorformlingen (19) nach Anspruch 1,
**dadurch gekennzeichnet, dass**
die zu sterilisierenden Vorformlinge (19), Vorformlinge für Behältnisse für Getränke und/oder andere fluide Medien sind.

3. Vorrichtung (50) zum Sterilisieren von Vorformlingen (19) nach Anspruch 1 oder 2,
**dadurch gekennzeichnet, dass**
das der Transportpfad zumindest in einem Abschnitt, in welchem die erste Außenbeaufschlagungseinrichtung (10) zum Sterilisieren zumindest eines Abschnitts einer Außenwandung von Vorformlingen (19) angeordnet ist, gekrümmt ist.

4. Vorrichtung (50) zum Sterilisieren von Vorformlingen (19) nach wenigstens einem der vorangegangenen Ansprüche,
**dadurch gekennzeichnet, dass**
auf der der ersten Außenbeaufschlagungseinrichtung (10) gegenüberliegenden Seite des Transportpfades eine weitere Außenbeaufschlagungseinrichtung (11) angeordnet ist, wobei die Außenbeaufschlagungseinrichtung (10) und die weitere Außenbeaufschlagungseinrichtung (11) bevorzugt entlang des Transportpfades versetzt angeordnet sind.

5. Vorrichtung (50) zum Sterilisieren von Vorformlingen (19) nach wenigstens einem der vorangegangenen Ansprüche,
**dadurch gekennzeichnet, dass**
ein Bereich des Transportpfades, in welchem die erste Außenbeaufschlagungseinrichtung (10) angeordnet ist, zumindest abschnittsweise von einer Strahlungsabschirmungsvorrichtung (21) mit mindestens einer äußeren Strahlungsabschirmungseinrichtung (23) und einer inneren Strahlungsabschirmungseinrichtung (22) umgeben ist, durch welche eine von der ersten Außenbeaufschlagungseinrichtung (10) abgegebene Strahlung zumindest teilweise absorbierbar ist.

6. Vorrichtung (50) zum Sterilisieren von Vorformlingen (19) nach Anspruch 5,
**dadurch gekennzeichnet, dass**
die Strahlungsabschirmungsvorrichtung (21) einen während des Transports von Behältnissen entlang des Transportpfades stationären Anteil und einen anderen, während des Transports von Behältnissen entlang des Transportpfades gegenüber dem ersten Anteil relativbeweglichen Anteil (16), aufweist.

7. Vorrichtung (50) zum Sterilisieren von Vorformlingen (19) nach wenigstens einem der vorangegangenen Ansprüche,
**dadurch gekennzeichnet, dass**
die Innenbeaufschlagungseinrichtung (15) zum Sterilisieren zumindest eines Abschnitts einer Innenwandung von Vorformlingen (19) eine Beschleunigungseinrichtung mit einer geringeren Beschleunigungsspannung aufweist als eine Beschleunigungseinrichtung der ersten Außenbeaufschlagungseinrichtung (10).

8. Vorrichtung (50) zum Sterilisieren von Vorformlingen (19) nach wenigstens einem der vorangegangenen Ansprüche,
**dadurch gekennzeichnet, dass**
zumindest die erste Außenbeaufschlagungseinrichtung (10), bevorzugt auch eine weitere Außenbeaufschlagungseinrichtung (11) jeweils ein im Wesentlichen rechteckiges oder ovales Ladungsträgeraustrittsfenster (30) aufweist, wobei bevorzugt zumindest eine Längsachse des Ladungsträgeraustrittsfensters (30) gegenüber einer Längsachse des mit Ladungsträgern zu beaufschlagenden Vorformlings (19) geneigt ist.

9. Vorrichtung (50) zum Sterilisieren von Vorformlingen (19) nach wenigstens einem der Ansprüche 5 - 8,
**dadurch gekennzeichnet, dass**
ein Abschnitt (32) einer gegenüber dem ersten Anteil (23) der Strahlungsabschirmungsvorrichtung (21) relativbeweglichen Trägerplatte (24) zumindest zeitweise ein in einem Kanal (39) befindliches Dichtungs- und/oder Sterilisationsmedium kontaktiert, um einen sich zumindest abschnittsweise entlang des Transportpfades erstreckenden Reinraum abzudichten.

10. Vorrichtung (50) zum Sterilisieren von Vorformlingen (19) nach Anspruch 9,
**dadurch gekennzeichnet, dass**
der Abschnitt (32) der gegenüber dem ersten Anteil (23) der Strahlungsabschirmungsvorrichtung (21) relativbeweglichen Trägerplatte (24) zumindest zeitweise von dem in dem Kanal (39) befindlichen Dichtungs- und/oder Sterilisationsmedium trennbar ist, um, insbesondere zur Wartung und/oder Reinigung, einen Zugang zu dem sich entlang des Transportpfades erstreckenden Reinraum zu ermöglichen.

11. Vorrichtung (50) zum Sterilisieren von Vorformlingen (19) nach wenigstens einem der vorangegangenen Ansprüche,
**dadurch gekennzeichnet, dass**
die Vorrichtung (50) eine Hubvorrichtung (17) aufweist, mittels welcher die Behältnisse während deren Transport entlang des Transportpfades und während deren Außensterilisation entlang deren Längsrichtung bewegbar sind.

12. Anlage zum Behandeln von Vorformlingen (19),
**dadurch gekennzeichnet, dass**
die Anlage mindestens eine Vorrichtung (50) zum Sterilisieren von Vorformlingen (19) nach wenigstens einem der vorangegangenen Ansprüche aufweist, welche stromabwärts bezüglich einer Heizeinrichtung zum Erwärmen von Kunststoffvorformlingen und stromaufwärts einer Abfülleinrichtung, bevorzugt stromaufwärts einer Umformungseinrichtung für Vorformlinge (19) angeordnet ist.

13. Verfahren zum Sterilisieren zumindest eines Abschnitts einer Innenwandung und eines Abschnitts einer Außenwandung von Vorformlingen (19) mittels beschleunigter Ladungsträger, wobei der Vorformling(19) durch mindestens eine erste Außenbeaufschlagungseinrichtung (10) zum Sterilisieren zumindest eines Abschnitts einer Außenwandung von Vorformlingen (19) und eine Innenbeaufschlagungseinrichtung (15) zum Sterilisieren zumindest eines Abschnitts einer Innenwandung von Vorformlingen (19) mit Ladungsträgern beaufschlagt wird, wobei die Innenbeaufschlagungseinrichtung (15) zumindest abschnittsweise durch eine Öffnung in den Vorformling (19) eingeführt wird, um eine Innenwandung des Vorformlings (19) mit austretenden Ladungsträgern zu beaufschlagen, wobei die Vorformlinge (19) während deren Sterilisation mit einer Transporteinrichtung (2, 4) entlang eines Transportpfades transportiert werden,
**dadurch gekennzeichnet, dass**
entlang eines Abschnitts des Transportpfades der Vorformlinge (19) zwischen der ersten Außenbeaufschlagungseinrichtung (10) und der Innenbeaufschlagungseinrichtung (15) eine Abstandsänderungseinrichtung (3), angeordnet ist, welche einen Abstands zwischen zwei entlang des Transportpfades aufeinander folgenden Vorformlingen (19) verändert.

14. Verfahren nach Anspruch 13,
**dadurch gekennzeichnet, dass**
Vorformlinge für Getränkebehältnisse und/oder andere fluide Medien sterilisiert werden.

15. Verfahren nach Anspruch 13 oder 14,
**dadurch gekennzeichnet, dass**
Vorformlinge (19) entlang eines Transportpfads transportiert werden, welcher zumindest in einem Abschnitt, in welchem die Außenbeaufschlagungseinrichtung (10) zum Sterilisieren zumindest eines Abschnitts einer Außenwandung von Vorformlingen (19) angeordnet ist, gekrümmt ist.

## Claims

1. Apparatus (50) for sterilising at least a section of an inner wall and a section of an outer wall of preforms (19) by means of accelerated charge carriers, with at least one first external application device (10) for sterilising at least a section of an outer wall of preforms (19) and an internal application device (15) for sterilising at least a section of an inner wall of preforms (19), wherein the internal application device (15) at least in sections can be introduced through an opening into the preform (19) in order to apply the emitted charge carriers to an inner wall of the preform (19), with a transport device (2, 4) by means of which the preforms can be transported along a transport path during their sterilisation, **characterised in that** along a section of the transport path of the preforms (19) between the first external application device (10) and the internal application device (15) is arranged a distance change device (3) to change a distance between two preforms (19) succeeding each other along the transport path.

2. Apparatus (50) for sterilising preforms (19) according to claim 1, **characterised in that** the preforms (19) to be sterilised are preforms for containers for beverages and/or fluid media.

3. Apparatus (50) for sterilising preforms (19) according to claim 1 or 2, **characterised in that** the transport path is curved at least in a section in which is arranged the first external application device (10) for sterilising at least a section of an outer wall of preforms (19).

4. Apparatus (50) for sterilising preforms (19) according to at least one of the preceding claims, **characterised in that** on the side of the transport path opposite the first external application device (10) is arranged a further external application device (11), wherein the external application device (10) and the further external application device (11) are arranged preferably offset along the transport path.

5. Apparatus (50) for sterilising preforms (19) according to at least one of the preceding claims, **characterised in that** a region of the transport path in which the first external application device (10) is arranged is surrounded at least in sections by a radiation screening apparatus (21) with at least an outer radiation screening device (23) and an inner radiation screening device (22), by means of which a radiation emitted by the first external application device (10) can be at least partly absorbed.

6. Apparatus (50) for sterilising preforms (19) according to claim 5, **characterised in that** the radiation screening apparatus (21) comprises a portion stationary during transport of the containers along the transport path and another portion mobile in relation to the first portion during transport of containers along the transport path.

7. Apparatus (50) for sterilising preforms (19) according to at least one of the preceding claims, **characterised in that** the internal application device (15) for sterilising at least a section of an inner wall of preforms (19) comprises an acceleration device with a lower acceleration voltage than an acceleration device of the first external application device (10).

8. Apparatus (50) for sterilising preforms (19) according to at least one of the preceding claims, **characterised in that** at least the first external application device (10), preferably also a further external application device (11), each comprises a substantially rectangular or oval charge carrier outlet window (30), wherein preferably at least one longitudinal axis of the charge carrier outlet window (30) is tilted in relation to a longitudinal axis of the preform (19) to be acted upon with charge carriers.

9. Apparatus (50) for sterilising preforms (19) according to at least one of claims 5 to 8, **characterised in that** a section (32) of a carrier plate (24) mobile in relation to the first portion (23) of the radiation screening device (21) at least for part of the time is in contact with a sealing and/or sterilisation medium present in a channel (39), in order to seal a clean room extending at least in sections along the transport path.

10. Apparatus (50) for sterilising preforms (19) according to claim 9, **characterised in that** the section (32) of the carrier plate (24) mobile in relation to the first portion (23) of the radiation screening device (21) can for at least part of the time be separated from the sealing and/or sterilisation medium present in the channel (39), in order to allow access to the clean room extending along the transport path, in particular for maintenance and/or cleaning.

11. Apparatus (50) for sterilising preforms (19) according to at least one of the preceding claims, **characterised in that** the apparatus (50) has a lift device (17) by means of which the containers can be moved along their longitudinal direction during their transport along the transport path and during their external sterilisation.

12. Plant for treating preforms (19) **characterised in that** the plant comprises at least one apparatus (50) for sterilising preforms (19) according to at least one of the preceding claims, which apparatus is arranged downstream of a heating device for heating plastics material preforms and upstream of a filling device, preferably upstream of a shaping device for preforms (19).

13. Method for sterilising at least a section of an inner wall and a section of an outer wall of preforms (19) by means of accelerated charge carriers, wherein the preform (19) is acted upon by charge carriers from at least one first external application device (10) for sterilising at least a section of an outer wall of preforms (19) and an internal application device (15) for sterilising at least a section of an inner wall of preforms (19), wherein the internal application device (15) at least in sections is introduced through an opening into the preform (19) in order to apply emitted charge carriers to an inner wall of the preform (19), wherein the preforms during their sterilisation are transported along a transport path by a transport device (2, 4), **characterised in that** along a section of the transport path of the preforms (19), between the first external application device (10) and the internal application device (15) is arranged a distance change device (3) which changes the distance between two preforms (19) succeeding each other along the transport path.

14. Method according to claim 13, **characterised in that** preforms are sterilised, for beverage containers and/or other fluid media.

15. Method according to claim 13 or 14, **characterised in that** preforms (19) are transported along a transport path which is curved at least in a section in which is arranged the external application device (10) for sterilising at least a section of an outer wall of preforms (19).

## Revendications

1. Système (50) pour la stérilisation d'au moins une section d'une paroi intérieure et d'au moins une section d'une paroi extérieure de préformes (19) au moyen de porteurs de charge accélérés, avec au moins un premier dispositif de sollicitation extérieure (10) pour la stérilisation d'au moins une section d'une paroi extérieure de préformes (19) et un dispositif de sollicitation intérieure (15) pour la stérilisation d'au moins une section d'une paroi intérieure de préformes (19), le dispositif de sollicitation intérieure (15) étant au moins en sections insérable dans la préforme (19) par une ouverture pour soumettre une paroi intérieure de la préforme (19) à des porteurs de charge sortants, avec un dispositif de transport (2, 4) au moyen duquel les préformes (19) peuvent être convoyées le long d'un chemin de transport pendant leur stérilisation,
**caractérisé en ce**
**qu'**un dispositif de variation d'intervalle (3) est disposé le long d'une section du chemin de transport des préformes (19) entre le premier dispositif de sollicitation extérieure (10) et le dispositif de sollicitation intérieure (15), pour modifier un intervalle entre deux préformes (19) successives le long du chemin de transport.

2. Système (50) pour la stérilisation de préformes (19) selon la revendication 1,
**caractérisé en ce que**
les préformes (19) à stériliser sont des préformes pour des récipients à boissons et/ou pour d'autres fluides.

3. Système (50) pour la stérilisation de préformes (19) selon la revendication 1 ou la revendication 2,
**caractérisé en ce que**
le chemin de transport est courbé sur au moins une section où est disposé le premier dispositif de sollicitation extérieure (10) pour la stérilisation d'au moins une section d'une paroi extérieure de préformes (19).

4. Système (50) pour la stérilisation de préformes (19) selon au moins une des revendications précédentes,
**caractérisé en ce**
**qu'**un autre dispositif de sollicitation extérieure (11) est disposé sur le côté du chemin de transport opposé au premier dispositif de sollicitation extérieure (10), le dispositif de sollicitation extérieure (10) et l'autre dispositif de sollicitation extérieure (11) étant préférentiellement disposés en décalage le long du chemin de transport.

5. Système (50) pour la stérilisation de préformes (19) selon au moins une des revendications précédentes,
**caractérisé en ce**
**qu'**une zone du chemin de transport où est disposé le premier dispositif de sollicitation extérieure (10), est au moins en sections entourée par une installation de protection contre les rayonnements (21) avec au moins un dispositif d'écran extérieur contre les rayonnements (23) et un dispositif d'écran intérieur contre les rayonnements (22), par lesquels un rayonnement émis par le premier dispositif de sollicitation extérieure (10) est au moins partiellement absorbable.

6. Système (50) pour la stérilisation de préformes (19) selon la revendication 5,
**caractérisé en ce que**
l'installation de protection contre les rayonnements (21) présente une partie fixe pendant le transport de récipients le long du chemin de transport et une autre partie (16) déplaçable par rapport à la première partie pendant le transport de récipients le long du chemin de transport.

7. Système (50) pour la stérilisation de préformes (19) selon au moins une des revendications précédentes,
**caractérisé en ce que**
le dispositif de sollicitation intérieure (15) pour la stérilisation d'au moins une section d'une paroi intérieure de préformes (19) comporte un dispositif d'accélération avec une tension d'accélération inférieure à celle d'un dispositif d'accélération du premier dispositif de sollicitation extérieure (10).

8. Système (50) pour la stérilisation de préformes (19) selon au moins une des revendications précédentes,
**caractérisé en ce**
**qu'**au moins le premier dispositif de sollicitation extérieure (10), préférentiellement aussi un autre dispositif de sollicitation extérieure (11), comportent chacun une fenêtre de sortie de porteurs de charge (30) sensiblement rectangulaire ou ovale, au moins un axe longitudinal de la fenêtre de sortie de porteurs de charges (30) étant préférentiellement incliné par rapport à un axe longitudinal de la préforme (19) à soumettre à des porteurs de charge.

9. Système (50) pour la stérilisation de préformes (19) selon au moins une des revendications 5 à 8,
**caractérisé en ce**
**qu'**une partie (32) d'un plateau support (24) mobile par rapport à la première partie (23) de l'installation de protection contre les rayonnements (21) entre au moins temporairement en contact avec un agent d'étanchéité et/ou de stérilisation se trouvant dans un canal (39), pour rendre étanche un espace stérile s'étendant au moins en sections le long du chemin de transport.

10. Système (50) pour la stérilisation de préformes (19) selon la revendication 9,
**caractérisé en ce que**
la section (32) du plateau support (24) mobile par rapport à la première partie (23) de l'installation de protection contre les rayonnements (21) est au moins temporairement séparable de l'agent d'étanchéité et/ou de stérilisation se trouvant dans le canal (39), pour permettre un accès à l'espace stérile s'étendant le long du chemin de transport, en particulier en cas de nettoyage et/ou d'entretien.

11. Système (50) pour la stérilisation de préformes (19) selon au moins une des revendications précédentes,
**caractérisé en ce que**
le système (50) comporte un dispositif de levage (17) au moyen duquel les récipients sont déplaçables pendant leur transport le long du chemin de transport et pendant leur stérilisation extérieure dans le sens de leur longueur.

12. Installation pour le traitement de préformes (19),
**caractérisée en ce que**
ladite installation comprend au moins un système (50) pour la stérilisation de préformes (19) selon au moins une des revendications précédentes, lequel est situé en aval d'un dispositif de chauffage de préformes en matière plastique et en amont d'un dispositif de remplissage, préférentiellement en amont d'un dispositif de transformation de préformes (19).

13. Procédé pour la stérilisation d'au moins une section d'une paroi intérieure et d'au moins une section d'une paroi extérieure de préformes (19) au moyen de porteurs de charge accélérés, la préforme (19) étant soumise à des porteurs de charge d'au moins un premier dispositif de sollicitation extérieure (10) pour la stérilisation d'au moins une section d'une paroi extérieure des préformes (19) et d'un dispositif de sollicitation intérieure (15) pour la stérilisation d'au moins une section d'une paroi intérieure des préformes (19), ledit dispositif de sollicitation intérieure (15) étant inséré au moins en sections dans la préforme (19) par une ouverture pour soumettre une paroi intérieure de la préforme (19) à des porteurs de charge sortants, les préformes (19) étant convoyées le long d'un chemin de transport par un dispositif de transport (2, 4) pendant leur stérilisation,
**caractérisé en ce**
**qu'**un dispositif de variation d'intervalle (3) est disposé le long d'une section du chemin de transport des préformes (19) entre le premier dispositif de sollicitation extérieure (10) et le dispositif de sollicitation intérieure (15), lequel modifie un intervalle entre deux préformes (19) successives le long du chemin de transport.

14. Procédé selon la revendication 13,
**caractérisé en ce que**
des préformes pour des récipients à boissons et/ou pour d'autres fluides sont stérilisées.

15. Procédé selon la revendication 13 ou 14,
**caractérisé en ce que**
des préformes (19) sont convoyées le long d'un chemin de transport, lequel est courbe au moins sur une section où est disposé le dispositif de sollicitation extérieure (10) pour la stérilisation d'au moins une section d'une paroi extérieure de préformes (19).
